# EUROPEAN PATENT SPECIFICATION

(11) **EP 1 530 972 B1**
(45) Date of publication and mention of the grant of the patent: **01.05.2013**
(21) Application number: 04256814.7
(22) Date of filing: 04.11.2004
(51) Int. Cl.: A61K 35/56, C12Q 1/68, G01N 33/50

(54) **Method for screening parasite preparations for efficiency in prevention and control of diseases**
Verfahren zum Auswählen von aus Parasiten hergestellten Preparationen zur Vorbeugung und Kontrolle von Krankheiten
Procédé pour trier des compositions préparés d'un parasite pour la prévention et le contrôle de maladies

(30) Priority: 17.11.2003 US 715659
(43) Date of publication of application: 18.05.2005
(73) Proprietor: University of Iowa Research Foundation Inc., Iowa City, IA 52242 (US)
(72) Inventor: Weinstock, Joel, Iowa City, Iowa 52246 (US); Elliott, David, North Liberty, IA 52317-9329 (US)
(74) Representative: Soames, Candida Jane

(56) References cited:
- WO-A-00/25810
- WO-A-01/13960
- WO-A-03/006058
- WO-A1-02/094228
- DE-A1- 10 163 602
- LEDINGHAM D L ET AL: "PROLONGATION OF RAT KIDNEY ALLOGRAFT SURVIVAL BY NEMATODES1,2" TRANSPLANTATION, WILLIAMS AND WILKINS, BALTIMORE, MD, US, vol. 61, no. 2, 27 January 1996 (1996-01-27), pages 184-188, XP000577116 ISSN: 0041-1337
- TERRAZAS L I ET AL: "The schistosome oligosaccharide lacto-N-neotetraose expands Gr1(+) cells that secrete anti-inflammatory cytokines and inhibit proliferation of naive CD4(+) cells: a potential mechanism for immune polarization in helminth infections." JOURNAL OF IMMUNOLOGY (BALTIMORE, MD. : 1950) 1 NOV 2001, vol. 167, no. 9, 1 November 2001 (2001-11-01), pages 5294-5303, XP002430365 ISSN: 0022-1767
- YAZDANBAKHSH MARIA ET AL: "Allergy, parasites, and the hygiene hypothesis." SCIENCE (NEW YORK, N.Y.) 19 APR 2002, vol. 296, no. 5567, 19 April 2002 (2002-04-19), pages 490-494, XP002430374 ISSN: 1095-9203
- ELLIOTT DAVID E ET AL: "INTESTINAL HELMINTHS INHIBIT COLITIS VIA INDUCTION OF REGULATORY CELLS" DIGESTIVE DISEASE WEEK ABSTRACTS AND ITINERARY PLANNER, XX, XX, 17 May 2003 (2003-05-17), page ABSTRACT467, XP001249096
- HORI SHOHEI ET AL: "Control of regulatory T cell development by the transcription factor Foxp3." SCIENCE (NEW YORK, N.Y.) 14 FEB 2003, vol. 299, no. 5609, 14 February 2003 (2003-02-14), pages 1057-1061, XP002430366 ISSN: 1095-9203
- MARON R ET AL: "Oral administration of schistosome egg antigens and insulin B-chain generates and enhances Th2-type responses in NOD mice." CLINICAL IMMUNOLOGY AND IMMUNOPATHOLOGY APR 1998, vol. 87, no. 1, April 1998 (1998-04), pages 85-92, XP002430368 ISSN: 0090-1229
- SHI H N ET AL: "T helper cell subclasses and clinical disease states" CURRENT OPINION IN GASTROENTEROLOGY 2002 UNITED STATES, vol. 18, no. 6, 2002, pages 711-716, XP009082500 ISSN: 0267-1379
- ANDERSON MICHELE K ET AL: "Definition of regulatory network elements for T cell development by perturbation analysis with PU.1 and GATA-3." DEVELOPMENTAL BIOLOGY 1 JUN 2002, vol. 246, no. 1, 1 June 2002 (2002-06-01), pages 103-121, XP002430370 ISSN: 0012-1606
- MIZOBUCHI TERUAKI ET AL: "Differential expression of Smad7 transcripts identifies the CD4+CD45RChigh regulatory T cells that mediate type V collagen-induced tolerance to lung allografts." JOURNAL OF IMMUNOLOGY (BALTIMORE, MD. : 1950) 1 AUG 2003, vol. 171, no. 3, 1 August 2003 (2003-08-01), pages 1140-1147, XP002430371 ISSN: 0022-1767
- WEIGMANN BENNO ET AL: "T-bet as a possible therapeutic target in autoimmune disease." EXPERT OPINION ON THERAPEUTIC TARGETS DEC 2002, vol. 6, no. 6, December 2002 (2002-12), pages 619-622, XP009082557 ISSN: 1744-7631
- ELLIOTT DAVID E ET AL: "Heligmosomoides polygyrus inhibits established colitis in IL-10-deficient mice." EUROPEAN JOURNAL OF IMMUNOLOGY OCT 2004, vol. 34, no. 10, October 2004 (2004-10), pages 2690-2698, XP002430367 ISSN: 0014-2980

## Description

### FIELD OF THE INVENTION

This disclosure relates to methods utilising helminthic parasite preparations.

### BACKGROUND OF THE INVENTION

Parasites are living entities that dwell on or in other creatures during some part of their life cycles, drawing nourishment from the host. Parasites that inhabit the intestines have a complex interplay with the mucosal immune system. They must establish a tranquil relationship with host mucosal defenses to survive.

Dysregulation of the immune system leading to an abnormal Th1 or Th2 response may be the cause of several human diseases. Some diseases due to dominant Th1 responses include IBD, rheumatoid arthritis, sarcoidosis, multiple sclerosis, and insulin-dependent diabetes melitis. Th2 related diseases include allergic diseases and cancer.

Recently, parasite exposure has been shown to reduce the severity of Experimental autoimmune encephalomyelitis (EAE) (Sewell et al., 2003, International Immunology, 15: 59-69). Experimental autoimmune encephalomyelitis (EAE) is an animal model for multiple sclerosis (MS) characterized by chronic inflammatory demyelination of the central nervous system (CNS). Sewell reported that in mice with *Schistosoma mansoni* ova immunization, the severity of EAE is reduced as measured by decreased clinical scores and CNS cellular infiltrates. There is a decreased IFN-gamma and increased IL-4, transforming growth factor-beta and IL-10 levels in the periphery, and the frequency of IL-4 producing neuroantigen-specific T cells increases in the brain.

Crohn's disease results from dysregulated T helper (Th)1 -type mucosal inflammation. Crohn's disease is rare in tropical countries but prevalent in developed countries with temperate climates, in which its incidence rose after 1940. In contrast, exposure to helminthic parasites is common in tropical countries but is rare in developed countries. Elliott et al. (2003, Am. J. Physiol. Gastrointest. Liver Physiol., 284:G385-G391) demonstrated that helminths may attenuate excessive Th1-type inflammation. To test that hypothesis, mice were exposed to eggs of the helminth Schistosoma mansoni and then challenged rectally with trinitrobenzesulfonic acid (TNBS) to induce colitis. Schistosome egg exposure attenuated TNBS colitis and protected mice from lethal inflammation. Schistosome egg exposure diminished IFN-gamma and enhanced IL-4 production from anti-CD3-stimulated spleen and mesenteric lymph node cells of TNBS-treated mice. Schistosome egg exposure decreased colonic IFN-gamma but increased IL-10 mRNA expression in TNBS-treated mice. Intact signal transducer and activator of transcription 6 was required for attenuation of colitis. The authors showed that exposure to helminths can decrease murine colonic inflammation.

de Jong et al. (2002, J. Immunol., 168:1704-1709) demonstrated protein extract derived from the helminth Schistosoma mansoni induced the development of DC2s that promote the development of Th2 cells via the enhanced expression of OX40 ligand. Likewise, toxin from the extracellular bacterium Vibrio cholerae induced development of DC2s as well, however, via an OX40 ligand-independent, still unknown mechanism. In contrast, toxin from the intracellular bacterium Bordetella pertussis induced the development of DC1s with enhanced IL-12 production, which promotes a Th1 cell development. Poly(I:C) (dsRNA, mimic for virus) induced the development of extremely potent Th1-inducing DC1 without an enhanced IL-12 production.

U.S. Patent Application 2003/0103938 A1 discloses a pharmaceutical composition consisting of IL-4 and SDF-1α, or IL-2 and SDF-1α for preventing or treating a Th1 cell- or Th2-cell-related disease in a human or an animal by modulating the Th1/Th2 ratio.

Doetze et al. (2000, International Immunology, 12: 623-630) investigated T cell proliferative hyporesponsiveness to Ov antigen (OvAg) by peripheral blood mononuclear cells (PBMC) from individuals with chronic helminth infections, i.e. generalized onchocerciasis (GEO), compared to PBMC from putatively immune (PI) individuals. In this study, mechanisms mediating this cellular hyporesponsiveness in GEO were investigated: the low proliferative response in PBMC from GEO individuals was associated with a lack of IL-4 production and significantly lower production of IL-5 compared to those from PI individuals, arguing against a general shift towards a T(h)2 response being the cause of hyporesponsiveness. In contrast, IL-10 and transforming growth factor (TGF)-beta, two cytokines associated with a T(h)3 response, seemed to mediate hyporesponsiveness: PBMC from individuals with GEO produced significantly more IL-10, and T cell proliferative hyporesponsiveness in this group could be reversed by the addition of anti-IL-10 and anti-TGF-beta antibodies. The authors showed that hyporesponsiveness was specific for OvAg and not observed upon stimulation with related nematode antigens, arguing for a T cell-mediated, Ov-specific down-regulation. Ov-specific T cells could be cloned from GEO PBMC which have a unique cytokine profile (no IL-2 but high IL-10 and/or TGF-beta production), similar to the T cell subsets known to suppress ongoing inflammation (T(h)3 and T(r)1), indicating that this cell type which has not been found so far in infectious diseases may be involved in maintaining Ov-specific hyporesponsiveness.

Th3 cells are a subset of regulatory T cells and were originally generated and identified in mice orally tolerized to MBP and suppressed the induction of experimental auimmune encephalomyelitis (EAE) by a TGF-b-dependent mechanism (Chen et al., 1994, Science, 265:1237-1240). Recent studies suggest that regulatory T cells can be induced against bacterial, viral and parasite antigens in vivo and might prevent autoimmune disease, infection-induced immunopathology or prolong pathogen persistence by suppressing protective Th1 responses (see, for example, McGuirk and Mills, 2002, Trends in Immunology, 23:450-455; Tung et al., 2001, Immunological Reviews, 182:135-148; and Maloy and Powrie, 2001, Nature Immunology, 2:816-822).

Regulatory T cells are generally described as lymphocytes that suppress immune responses, i.e., both antibody-mediated and/or cell-mediated (e.g., for reviews, see McGuirk P, Mills KH. Pathogen-specific regulatory T cells provoke a shift in the Th1/Th2 paradigm in immunity to infectious diseases. Trends Immunol 2002;23(9):450-5; Field, A. C., L. Caccavelli, M. F. Bloch, and B. Bellon. 2003. Regulatory CD8+ T cells control neonatal tolerance to a Th2-mediated autoimmunity. Journal of Immunology. 170:2508-2515; von Herrath MG, Harrison LC. Antigen-induced regulatory T cells in autoimmunity. Nat Rev Immunol. 2003 Mar;3(3):223-32; Francois Bach J. Regulatory T cells under scrutiny. Nat Rev Immunol. 2003 Mar;3(3):189-98; Curotto de Lafaille MA, Lafaille JJ. CD4(+) regulatory T cells in autoimmunity and allergy. Curr Opin Immunol. 2002 Dec;14(6):771-8; McGuirk P, Mills KH. Pathogen-specific regulatory T cells provoke a shift in the Th1/Th2 paradigm in immunity to infectious diseases. Trends Immunol. 2002 Sep;23(9):450-5; Tung KS, Agersborg SS, Alard P, Garza KM, Lou YH. Regulatory T-cell, endogenous antigen and neonatal environment in the prevention and induction of autoimmune disease. Immunol Rev. 2001 Aug;182:135-48; Read S, Powrie F. CD4(+) regulatory T cells. Curr Opin Immunol. 2001 Dec; 13(6):644-9; Yssel H, Lecart S, Pene J. Regulatory T cells and allergic asthma. Microbes Infect. 2001 Sep;3(11):899-904). These regulatory T cells (Tr cells) express a transmembrane protein (called CD25) that is alpha chain of the receptor for interleukin-2 (IL-2). Like other T cells, they also express the αβ (alpha-beta) T-cell receptor for antigen (TCR) and can only be activated if it binds to the peptide-class II MHC molecule, or in the case of CD8 regulatory cells Class I MHC, for which it is specific. However, if activated, they begin to secrete large amounts of interleukin 10 (IL-10) and often some transforming growth factor-beta (TGF-β) as well. Both these lymphokines are powerful immunosuppressants inhibiting Th1 help for cell-mediated immunity and inflammation, Th2 help for antibody production, and, possibly, the action of CD8⁺ cytolytic T lymphocytes (CTL). Some other regulatory T cells differ from Tr cells. For example, Tr1 Cells resemble Tr cells in several ways, although they do not express large amounts of CD25 on their surface. They require IL-10 for their formation and, once mature, secrete large amounts of it. The main lymphokine for Th3 Cells is TGF-β.

Transplantation 61, 2 (1996) describes the use of preparations containing *Nippostrongylus brasiliensis* organism or an extract thereof in the treatment of allograft survival.

WO00/25810 describes the use of a protein obtained from the nematode *Heligmosomoides polygyrus* for immunosuppressive treatment and as an anti-allergicum.

Journal of Immunology 167, 9 (2001) discloses that a helminths inhibits colitis in a model of inflammatory bowel disease by induction of regulatory T-cells.

*Digestive disease week abstracts and itinerary planner* May (2003) describes that *Heligmosomoides polygyrus* inhibits colitis in a model of inflammatory bowel disease by inducing regulatory T cells.

WO02/094228 describes helminthic compositions in the prevention and treatment of allergies and asthma.

Science 296, 5567 (2002) reviews the hygiene hypothesis in relation to parasites and allergies.

WO03/006058 describes methods and compositions for the identification of novel targets for diagnosis, prognosis, therapeutic intervention and prevention of autoimmune disorders, transplant rejection and cancer.

Science 299, 5609 (2003) describes the control of regulatory T-cell development by the transcription factor *Foxp3.*

Developmental Biology 246 1 (2002) describes the definition of regulatory network elements for T cell development by perturbation analysis with PU.1 and GATA-3.

Journa/ of Immunology 171, 3 (2003) describes that the differential expression of *Smad7* transcripts identifies the CD4⁺CD45RC^{high} regulatory T cells that mediate Type V collagen-induced tolerance to lung allografts.

Expert Opinion on Therapeutic Targets 6, 6 (2002) describes T-bet as a possible therapeutic target in autoimmune disease.

WO01/13960 describes *in vivo* gene therapy methods for the treatment of joint diseases, such as osteoarthritis.

It remains unknown whether regulatory T cells play a role in the prevention or treatment of Th1 or Th2 related diseases using a helminthic parasite preparation.

### SUMMARY OF THE INVENTION

The aspects and embodiments of the present invention are set forth in the claims.

The present disclosure is based on the finding that parasite preparations can alter the activity of regulatory T cells in the treatment of Th1 or Th2 related disease.

The present disclosure provides a method of screening a helminthic parasite preparation that alters a regulatory T cell activity, the method comprising the steps of: (a) obtaining a helminthic parasite preparation; (b) contacting the helminthic parasite preparation with a target; and (c) determining the level of an internal marker for regulatory T cell activity in the target after the contacting, where a change in the level of the internal marker after the contacting is indicative of the helminthic parasite preparation altering a regulatory T cell activity.

The present disclosure also provides a method of screening a helminthic parasite preparation that alters a regulatory T cell activity, the method comprising the steps of: (a) obtaining a helminthic parasite preparation; (b) contacting the helminthic parasite preparation with a target; and (c) determining the level of a cell surface marker for regulatory T cell in the target after the contacting, where a change in the level of the cell surface marker after the contacting is indicative of the helminthic parasite preparation altering a regulatory T cell activity.

The present disclosure also provides a method for treating an animal with a Th1 or Th2 related disease by administering a helminthic parasite preparation that alters a regulatory T cell activity to the animal.

The present disclosure also provides a method for monitoring the treatment efficacy of a helminthic parasite preparation for an autoimmune or allergy disease in an animal comprising: (a) administering a composition comprising a helminthic parasite preparation or a fraction thereof to the animal; and (b) determining the level of a regulatory T cell activity in the animal after the administering, where an increase in the level of the regulatory T cell activity after the administering is indicative of the treatment efficacy of the helminthic parasite preparation.

The disease being treated may be one selected from the group consisting of: Inflammatory bowel disease, Rheumatoid arthritis, Lupus, Juvenile Insulin-dependent Diabetes Mellitus (Type 1), Sarcoidosis, Multiple Sclerosis, Asthma and allergic rhinitis.

The regulatory T cell marker may be an internal marker, a cell surface marker, or a secreted marker.

Preferably, the internal marker is Scurfin, Smad7, Gata3, Tbet (Tbx21).

Also preferably, the cell surface marker is selected from the group consisting of: CD4, CD45RB^{lo}, CD45Rc, Cytolytic T lymphocyte associated antigen 4 (CTLA-4), CD25, CD103, CD62L, α_{E}β integrin, latency-associated peptide (LAP) or glucocorticoid induced TNF receptor family related protein (GITR), Experimental allergic encephalitis (EAE), chemokine receptor CCR5, TI-ST2.

Also preferably, the secreted marker is IL-5, IL-10 or TGFβ.

### BRIEF DESCRIPTION OF THE DRAWINGS

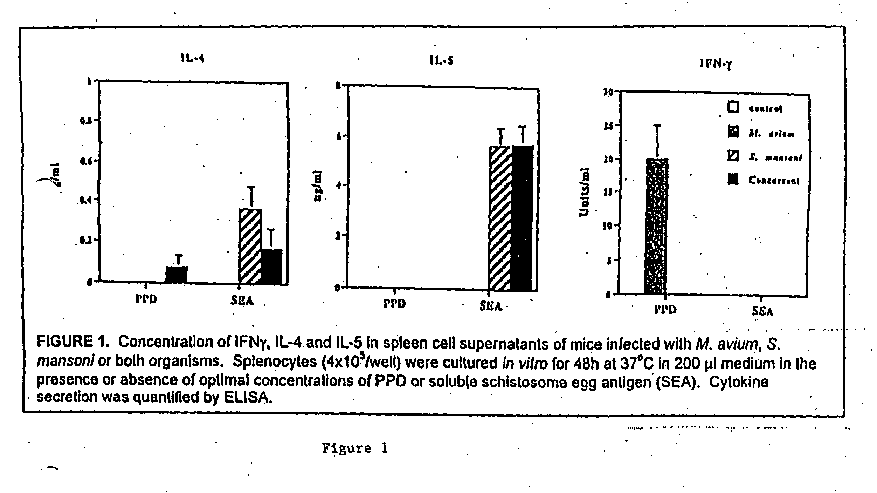

Figure 1. The figure describes the concentrations of IFNγ, IL-4 and IL-5 measured in spleen cell supernatants of mice infected with *M. avium, S. mansoni* or both organisms according to one embodiment of the disclosure. Splenocytes (4 x 10⁵/well) were cultured *in vitro* for 48 h at 37°C in 200 µl of medium in the presence or absence of optimal concentrations of PPD or SEA. Cytokine secretion was quantified by ELISA.

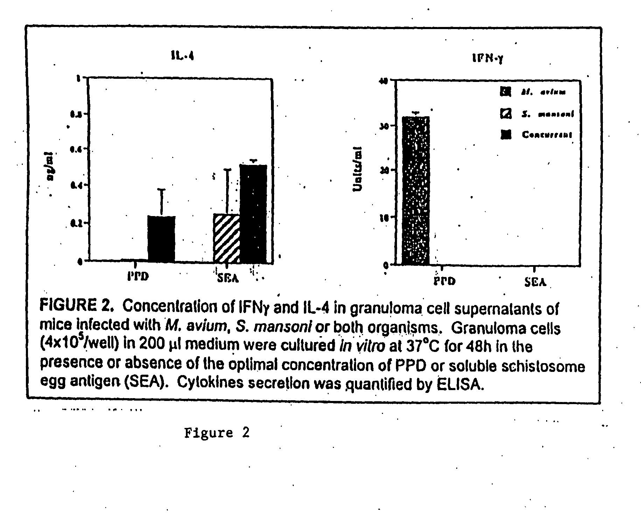

Figure 2. The figure describes the concentrations of IFNγ and IL-4 in granuloma cell supernatants of mice infected with *M. avium, S. mansoni* or both organisms according to one embodiment of the disclosure. Granuloma cells (4 x 10⁵/well) in 200 µl of medium were cultured *in vitro* at 37°C for 48 h in the presence or absence of the optimal concentration of PPD or SEA. Cytokines were quantified by ELISA.

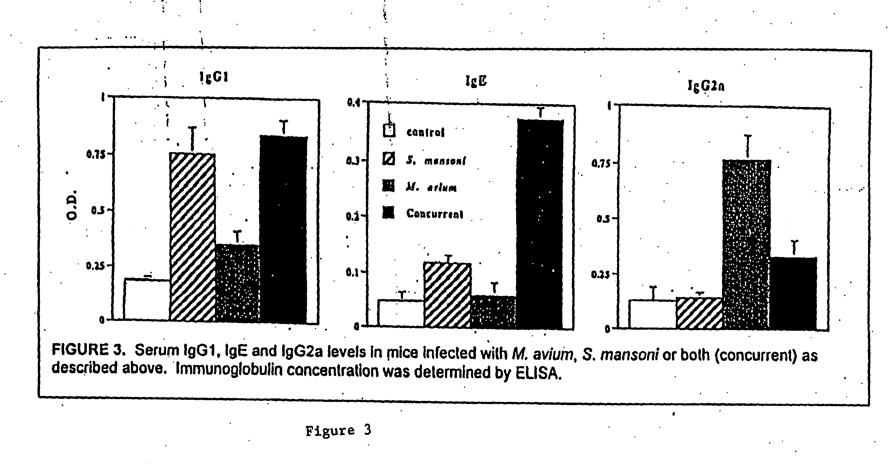

Figure 3. The figure describes the serum IgGI, IgE and IgG2a levels measured in mice infected with *M. avium, S. mansoni* or both (concurrent) according to one embodiment of the disclosure. Immunoglobulin concentration was determined by ELISA.

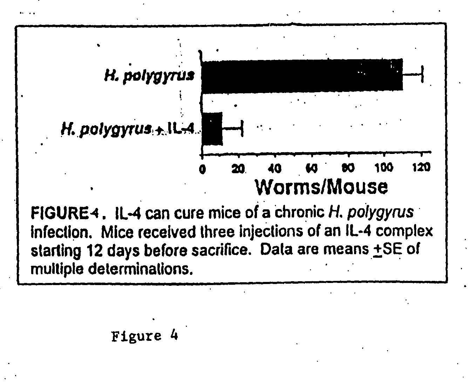

Figure 4. The figure shows that IL-4 treatment can cure mice of a chronic *H. polygyrus* infection according to one embodiment of the disclosure. Mice received three injections of an IL-4 complex starting 12 days before killing. Data are means +/- SE of multiple determinations.

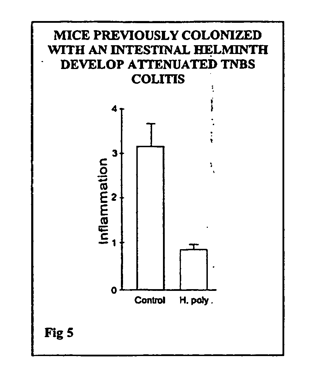

Figure 5: Data represent mean inflammatory score±SD from three separate experiments analyzing >4 WT mice/group.

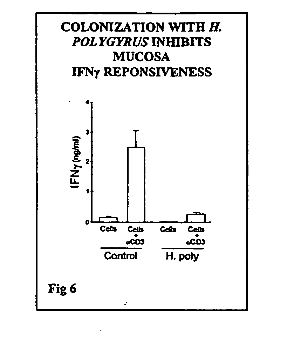

Figure 6: WT mice without IBD were colonized with *H. polygyrus*. Controls received sham gavage. LPMC were isolated from the TI and cultured for 48h *in vitro* with or without anti-CD3/anti-CD28 stimulation. IFNγ in supernatants was measure with ELISA. Data are mean ±SE of 6 determinations from 2 independent experiments.

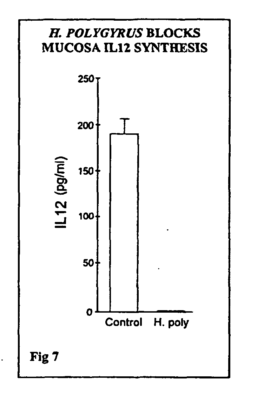

Figure 7: WT mice were treated as in Fig 2. Isolated LPMC were cultured 48h *in vitro* with CpG oligo to promote IL12 production. IL12 was measured with ELISA. Data are mean±SE of 6 determinations from 2 independent experiments.

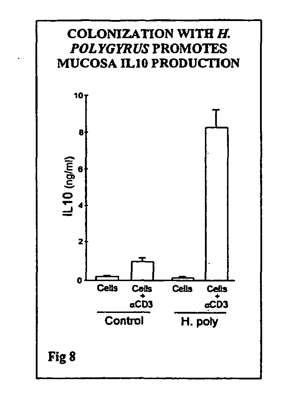

Figure 8: WT mice without IBD were colonized with *H. polygyrus*. Controls received sham gavage. LPMC were isolated and cultured 2 wks later as in Figure 2. Data are mean ±SE of 6 determinations from 2 independent experiments.

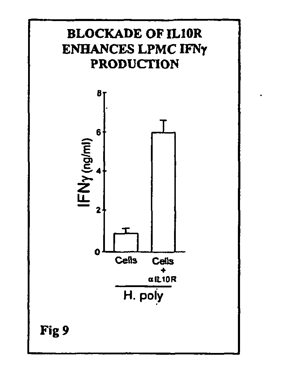

Figure 9: WT mice were colonized with *H. polygyrus*. LPMC were isolated and cultured for 48h *in vitro* with anti-CD3/anti-CD28 +/αIL10R mAb. IFNγ in supernatants was measure with ELISA. Data are mean ±SE of 3 independent determinations.

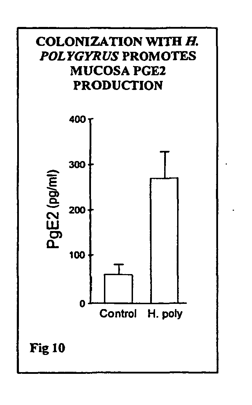

Figure 10: WT mice without IBD were colonized with *H. polygyrus*. Controls received sham gavage. LPMC were isolated and cultured 2 wks later as in Figure 2. Data are mean ±SE of 6 determinations from 2 independent experiments.

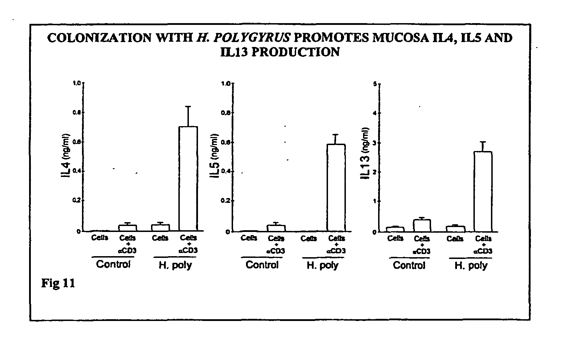

Figure 11: WT mice without IBD were colonized with *H. polygyrus*. Controls received sham gavage. LPMC were isolated and cultured 2 wks later as in Figure 2. Data are mean ±SE of 6 determinations from 2 independent experiments.

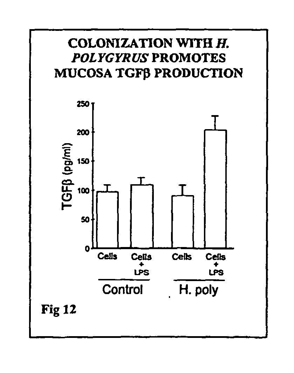

Figure 12: WT mice without IBD were colonized with *H. polygyrus*. Controls received sham gavage. LPMC were isolated and cultured 2 wks later as in Figure 2. LPMC were isolated from the TI and cultured for 48h *in vitro* with or without LPS stimulation. Data are mean ±SE of 9 determinations from 3 independent experiments.

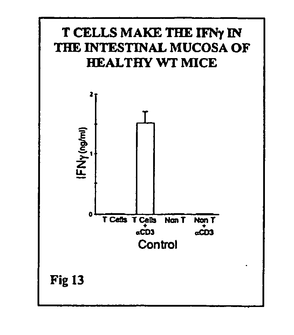

Figure 13: T cells were isolated form dispersed intestinal LPMC using magnetic beads. T cell-enriched (T cells) and T cell-depleted (non T) fractions were cultured 48h *in vitro* with or without anti-CD3/anti-CD28 stimulation before assaying IFNγ in supernatants. Data are mean ±SE of 6 determinations from 2 independent experiments.

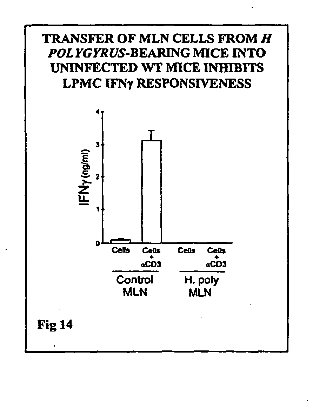

Figure 14: WT uninfected mice received 2x10⁷ MLN cells from mice colonized with *H. polygyrus*. One wk later LPMC were isolated from these recipients and cultured 48h with or with anti-CD3/anti-CD28 stimulation before assaying IFNγ in supernatants. Data are mean ±SE of 6 determinations from 2 independent experiments.

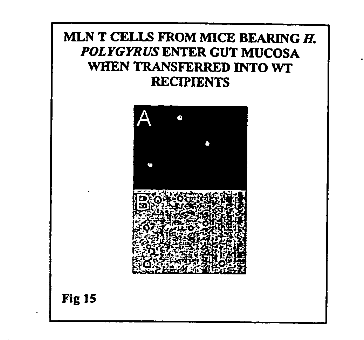

Figure 15: GFP WT mice were colonized with *H. polygyrus*. Two wks later, MLN cells from these helminth-conditioned GFP mice were transferred into normal C57BL/6 mice by I.P. injection. Seven days later, the lamina propria was dispersed and examined for GFP+ T cells under fluorescence microscopy. A) GFP+ LP T cells. B) Same field under light microscopy.

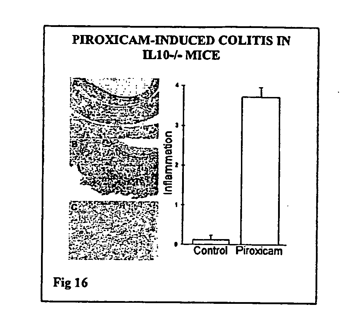

Figure 16: IL/10-/- animals received food containing piroxicam beginning wk-5 of age. After 2 wks of treatment, piroxicam was stopped and colitis was assessed 2 wks later. Age-matched IL10-/- controls received no piroxicam (Control). Colitis was scored in histological sections by blinded readers using a 0-4 point scale. A) Colon of a control IL10^{-/-} mouse. B) Colon of an age-matched IL10^{-/-} mouse after piroxicam treatment. C) Infiltration of the colonic lamina propria with CD4⁺ T cells following piroxicam (H&E 10X, IHC 20X) Graph data are means from 5 separate experiments, each with 5-6 mice/group.

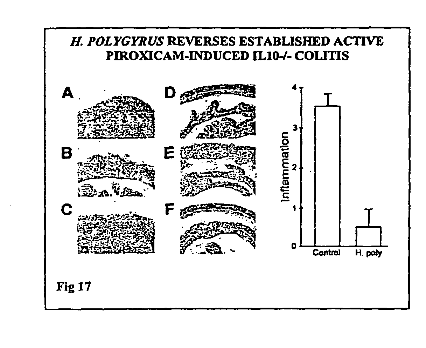

Figure 17: Panels A, B and C show the typical colonic inflammation in piroxicam-treated mice that did not receive *H. polygyrus*. D, E and F show the improvement in the colitis 2 wks after receiving *H. polygyrus* (All H&E, 4X). Colitis is scored on a 0-4 point scale. Data are means ±SE from 11 animals studied in 2 separate experiments.

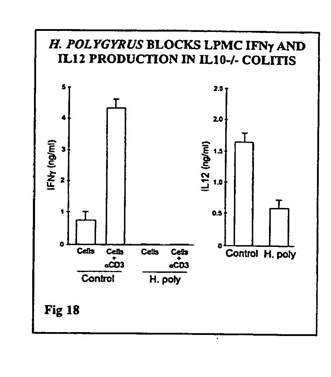

Figure 18: Colitic IL10-/- mice received *H*. *polygyrus* or sham gavage as described for Fig 12. Two wks later, LPMC were isolated and cultured 48h *in vitro* with or without anti-CD3/anti-CD28 stimulation (IFNγ) or with CpG oligo (IL12) Data are mean ±SE of 8-9 determinations from 3 independent experiments.

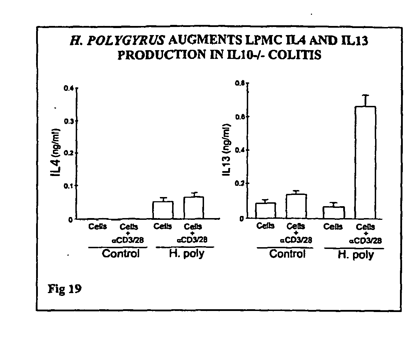

Figure 19: Colitic IL10^{-/-} mice were treated as in Fig 13. Data are means ±SE of 9 determinations from 3 independent experiments.

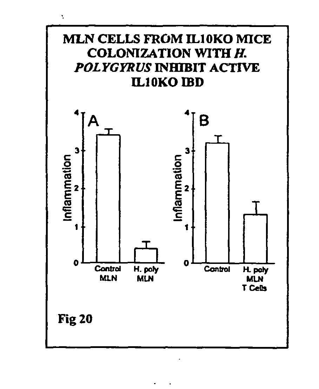

Figure 20: A) MLN cells isolated from IL10^{-/-} mice 2 wks after receiving *H. polygyrus* or sham (Control) gavage were transferred (20x10⁶) by i.p. injection into piroxicam-treated mice. Two wks after transfer colitis was assessed as in Fig 12. B) MLN T cells isolated from control or colonized mice were transferred (5x10⁶) to colitic mice. Data are means ± SE from 9 mice/group in 2 separate experiments.

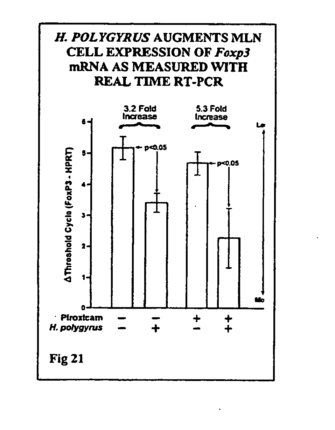

Figure 21: mRNA was extracted from MLN cells isolated from IL10^{-/-} mice 2 wks after sham or *H. polygyrus* gavage following 2wks of sham or piroxicam-treatment. Real-time PCR for *Foxp3* was normalized to HPRT mRNA (Methods). Note: Higher expression requires fewer PCR cycles to reach threshold. Data are means ± SE from 3 independent experiments each using 4 mice/group.

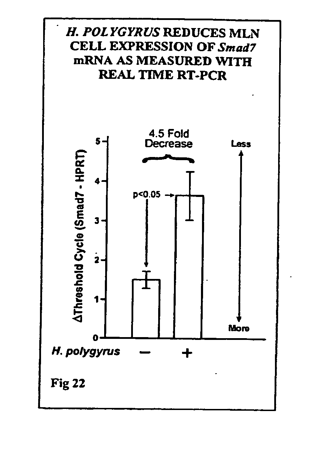

Figure 22: mRNA was extracted from MLN cells of IL10^{-/-} mice 2 wks after sham or *H. polygyrus* gavage. Lower expression requires more PCR cycles to reach threshold. Data are means ± SE from 3 independent experiments each using 4 mice/group.

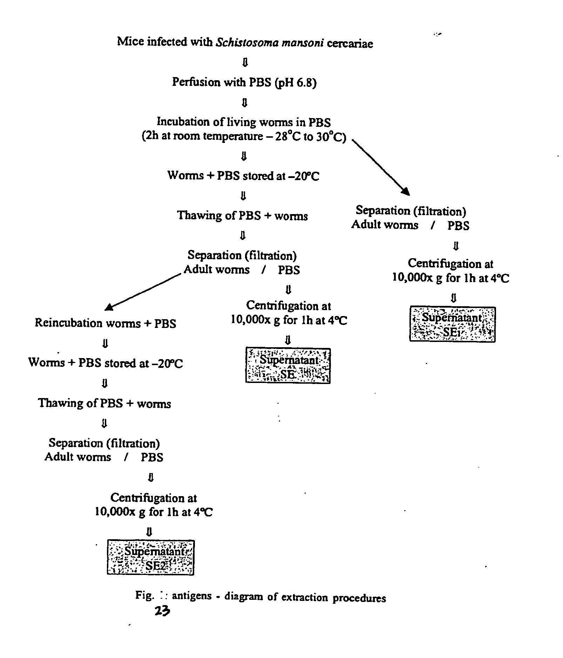

Figure 23. A schematic diagram for an antigen preparation procedure.

### DETAILED DESCRIPTION OF THE INVENTION

The invention encompasses an in vitro method of screening for a helminthic parasite preparation that alters a human regulatory T cell activity, as set forth in the claims.

As used herein, the term "helminth parasite preparation" includes, but is not limited to, any one of whole parasite, parasite extract, parasite ova, parasite ova extract, parasite egg, parasite egg extract, parasite larvae, parasite larvae extract, parasite cercariae and parasite cercariae extract. A "parasite preparation" may also be an isolated protein, polymcleotide carbohydrate or lipid derived from the parasite and its extract.

As used herein, the term "specific-pathogen free" applied to animals raised for use in the present invention when the animals are known to be free of a specific pathogenic microorganism that can cause disease in a target animal or human. Methods for raising pathogen free animals are known in the art, e.g., see references complied by Brown et al., A Bibliography on the Culture and Maintenance Specific Pathogen-Free Organisms, available at http://www.ctsa.org/upload/publicafion/CTSA_16631681202959092495.pdf Brown et al. provides references for raising different farm and laboratory animals in SPF environment, as well as procedures and requirements on building design and management. Also see M. Michael Swindle (J. Invest. Surg., 9:267-271, 1996 ) for SPF procedure and lists several potential human viral and bacterial pathogens of concern.

As used herein, the term "regulatory T cells" refers to a lymphocyte cell population which secrets at least 2-fold increase (e.g., 3-fold, 4-fold, 5-fold, 6-fold, 8-fold, 10-fold or more) of IL-10 and/or TGFβ, as compared to naive T cells. The determination of IL-10 or TGFβ secretion is known in the art. For example, it may be determined by culturing the cells in vitro for 24 or 48 with or without a T cell stimulant like anti-CD3 and then assaying the culture supernatant for these cytokines using cytokine specific ELISAs. In addition, regulatory T cells are also characterized by a high level of FoxP3 transcript as compared to other types of T cells (e.g., naive T cells). By "high level of FoxP3 transcript," it is referred to at least 4-fold increase in the level as compared to other types of T cells. FoxP3 is detectable by using real-time PCR or quantitative PCR (e.g., using PCR primers CCCAGGAAAGACAGCAACCTT, TTCTCACAACCAGGCCACTTG (SEQ ID NO. 1), and labeled probe 6FAM-ATCCTACCCACTGCTGGCAAATGGAGTC-TAMRA (SEQ ID NO. 2) as described in Hori, S., T. Nomura, and S. Sakaguchi. 2003. Control of regulatory T cell development by the transcription factor Foxp3. Science. 299:1057-1061). Values are normalized to HPRT expression, which is a housekeeping gene. Alternatively, FoxP3 protein product, Scurfin, can be detected by Western blotting analysis as known in the art, e.g., using Goat Anti-FoxP 3 (FoxP3) Polyclonal Antibody (Catalog Number ab2481, Novus Biologicals, Littleton, CO). Optionally, the regulatory T cells may also make much less IFNγ as compared to other T cells (e.g., naive T cells), i..e., at least 2-fold, preferably 3-fold, 4-fold, 5-fold, 6-fold, 7-fold, 8-fold, 10-fold or less. Also optionally, regulatory T cells also can be detected by using intracytoplasmic flow analysis to detect T cells expressing IL10 and/or TGFβ but little or no IFNγ. Additional optional markers as described herein below may also be used for detecting regulatory T cells or the activity of regulatory T cells.

As used herein, the term "naïve T cells" refers to T-cells arising from the immune system's production of fresh cells in the bone marrow. Naive T-cells respond to newly encountered pathogens containing antigens the immune system has not processed before. The naive T-cells may be identified according to methods known in the art (for reviews, see, e.g., Tough et al., 1999, Immunol Rev. 170:39-47; Itano et al., 2003, Nat Immunol. 4(8):733-9; Berard et al., 2002, Immunology. 106(2):127-38).

As used herein, the term "parasite preparation that alters a regulatory T cell activity" refers to a parasite preparation which changes the activity of regulatory T cell in a target by at least 40%, e.g., 50%, 80%, 100%, 2 fold, 4 fold, 6 fold, 8 fold, 10 fold, or more upon contacting the target, compared to the regulatory T cell activity in the target without contacting the parasite preparation. The activity of regulatory T cell may be measured by monitoring the level of a regulatory T cell internal marker (e.g., a transcription factor such as FoxP3 mRNA or its protein product Scurfin, Smad7, Gata3, or Tbet (Tbx21)), or a regulatory T cell surface marker (e.g., CD4, CD45RB^{lo}, CD45Rc, Cytolytic T lymphocyte associated antigen 4 (CTLA-4), Ox40, 4-1BB, CD25, CD103, CD62L, α_{E}β integrin, latency-associated peptide (LAP) or glucocorticoid induced TNF receptor family related protein (GITR), Experimental allergic encephalitis (EAE), chemokine receptor CCR5, TI-ST2) or a secreted marker (e.g., IL4, IL13, IL-5, IL-10, TGFβ). An increased regulatory T cell activity may be represented by an increase (e.g., FoxP3, Ox40, 4-1BB, CD4, CTLA-4, CD25, CD 103, CD62L, α_{E}β integrin' LAP, GITR, EAE, CCR5, TI-ST2, IL-10, TGFβ) or a decrease (e.g., D45Rc), in the level of a regulatory T marker, by at least 40%, e.g., 50%, 80%, 100%, 2 fold, 4 fold, 6 fold, 8 fold, 10 fold, or more, measured according to methods known in the art and as described herein.

A parasite preparation that alters a regulatory T cell activity may change the level of two or more markers described herein above.

As used herein, the term "the level of a regulatory T cell marker" refers to the amount of a specific regulatory T cell marker in a target, e.g., measured by micrograms or molar amounts. Typically, the regulatory T cell marker is a protein, for which the amount may be measured by any known methods in the art for protein quantitation, for example, by ELISA, western blot, immunoprecipitation, radioimmunoassay, or FACS analysis (e.g., in Innis et al., (1990) Academic Press, Inc.; Molecular Cloning, A Laboratory Manual (2d Edition, Sambrook, et al. (1989); and Current Protocols in Molecular Biology (1997, Ausubel et al., John Weley & Sons, Inc.). The level of a protein marker may also be measured at its mRNA level according to methods known in the art, e.g., by northern blot, quantitative RT-PCR, microarray analysis (e.g., in Innis et al., (1990) Academic Press, Inc.; Molecular Cloning, A Laboratory Manual (2d Edition, Sambrook, et al. (1989); and Current Protocols in Molecular Biology (1997, Ausubel et al., John Weley & Sons, Inc.).

As used herein, the term "target" refers to an in vitro system (e.g., cultured tissue or cells, transcription/translation extract).

The disclosure also describes a method of preventing or treating a disease caused by an altered immune response in an animal comprising administering a helminthic parasite preparation in an amount sufficient to prevent or treat the disease in the animal.

As used herein, the term "disease caused by an altered immune response" refers to a disease which an animal (e.g., a human) develops due to a difference in its immune response compared to a non-disease animal. For example, the disease animal may have an abnormal (e.g., excessive or non-sufficient) immune response to an antigen (e.g., a self or a non-self antigen) compared to a response to the same antigen in a normal non-disease animal. A "disease caused by an altered immune response", according to the present disclosure, includes, but is not limited to, a Th1 related disease or a Th2 related disease as described herein.

As used herein, the term "Th1 related disease" refers to a disease in which Th1 cells support, cause or mediate the disease process or in which Th1 cells are involved in curing or alleviating the symptoms of the disease, which may be represented by an enhanced or reduced Th1 activity. By "enhanced", it is meant that a diseased animal has an increase (e.g., at least 2-fold, and possible 5-fold, 6 fold, 8 fold, 10-fold, or more) in its Th1 activity, compared to another animal without the disease. An enhanced Th1 activity may be measured by an increase in the level of secreted cytokines (e.g., IL-2, IFN-γ, TNFα, IgG2a, IL-12, IL-18, IL-23), or a decrease in the level of secreted cytokines (e.g., IL-4, IL-13), according to methods known in the art. By "reduced", it is meant that a diseased animal has a decrease (e.g., at least 50% lower, or 2 fold, and possible 5-fold, 6 fold, 8 fold, 10-fold, or lower) in its Th1 activity, compared to another animal without the disease. A reduced Th1 activity may be measured by a decrease in the level of secreted cytokines (e.g., IFN-γ, TNFα, IgG2a), or an increase in the level of secreted cytokines (e.g., IL-4, IL-13), according to methods known in the art, and as described herein and in U.S. application US 2003/039666 and 09/362,598.

As used herein, the term "reduced" is used interchangeably with the term "decreased", and the term "increased" is used interchangeably with the term "enhanced."

As used herein, the term "Th2 related disease" refers to any disease, in which Th2 cells support, cause or mediate the disease process or in which Th2 cells are involved in curing or alleviating the symptoms of the diseases, which may be represented by an enhanced or reduced Th2 activity. By "enhanced", it is meant that a diseased n animal has an increase (e.g., at least 2-fold, and possible 5-fold, 6 fold, 8 fold, 10-fold, or more) in its Th2 activity, compared to another animal without the disease. An enhanced Th2 activity may be measured by an increase in the level of secreted cytokines and antibodies (e.g., IL-4, IL-5, IgE and IgG1) according to methods known in the art. By "reduced", it is meant that a diseased animal has a decrease (e.g., at least 50%, 2-fold, and possible 5-fold, 6 fold, 8 fold, 10-fold, or lower) in its Th2 response, compared to another animal without the disease. A reduced Th2 activity may be measured by a decrease in the level of secreted cytokines and antibodies (e.g., IL-4, IL-5, IgE and IgG1) according to methods known in the art, and as described herein and in U.S. application US 2003/039666.

As used herein, the term "treatment efficacy" refers to the effectiveness of a treatment of a disease using a parasite preparation. "Treatment efficacy" is usually measured by the clinical response to a specific disease for which the animal has been or is being treated with a parasite preparation.

### HELMINTHIC PARASITES

Useful helminthic parasites include, but are not limited to, two groups. The first group is helminthic parasites that naturally colonize humans and the second group is helminthic parasites that colonize animals, but may afford protection to humans.

In the first group, helminthic parasites are elaborate multicellular worms with complex life cycles and development. The nematodes (non-segmented round worms), and the platyhelminths (flat worms) are two groups of helminths that colonize the human intestines. In accordance with the present invention, any one of a number of helminth parasites that naturally colonize humans or animals will provide the intended results.

Nematodes that frequently inhabit the human gut are *Ascaris lumbricoides, Enterobius vermicularis* (pin worm), *Trichuris trichiura* (whipworm), *Ancylostoma duodenale and Necator americanus* (hookworms), and *Strongyloides stercoralis. Trichinella spiralis* infests the small intestine briefly.

The platyhelminths include the trematodes and cestodes. The most common adult trematodes that reside in the human intestines are *Fasciolopsis, Echinostoma* and *Heterophyes* species. Those that live in the biliary system include *Clonorchis sinensis, Opisthorchis viverrini and felineus,* and *Fasciola hepatica. Schistosoma* dwell in the venous system, but several species chronically affect the gut by the passage of eggs through the intestinal wall. Adult cestodes commonly infecting humans are *Diphyllobothrium* species (fish tapeworm), *Taenia saginata* (beef tapeworm), *Taenia solium* (pork tapeworm) and *Hymenolepsis nana* (dwarf tapeworm).

Other helminths of interest include the filarial parasites and the lung flukes. These do not have a gut phase, but stimulate strong Th2-type responses.

The second general group of helminthic parasites that can be utilized in the present invention include helminths that colonize animals, but may afford humans protection against immune-mediated diseases. These include *Trichuris muris* (mouse whipworm), *Trichinella spiralis, Nippostrongylus brasiliensis, Heligmonsomoides polygyrus* and *Hymenolepsis nana,* all of which are intestinal helminths that infect mice. Additionally, *Angiostrongylus* is a rat helminth. *Trichuris suis* and *Ascaris suum* are pig helminths that can infect humans. *Trichuris vulpis, Toxocara* species, *Gnathostoma,* and *Ancylostoma* are dog or cat helminths that also can infect humans. *Anisakis* and *Pseudoterranova* are nematodes of marine mammals that can transmit to humans. Bird schistosomes can transiently infect humans. Such schistosomes include *S. douthitti, Trichobilharzia ocellata, T. stagnicolae, T. physellae, and Gigantobilharzia huronensis*.

The helminthic preparation may be selected from the group consisting of helminiths that naturally colonize humans and helminths that colonize animals but may protect humans or animals from a disease caused by an altered immune response.

In some embodiments of the invention, the helminth parasite is a nematode, and may be selected from the group such as *Ascaris lumbricoides, Enterobius vermicularis, Trichuris trichiura, Ancylostoma duodenale* and *Necator americanus, Strongyloides stercoralis and Trichinella spiralis*.

In other embodiments of the invention, the helminthic parasite is a platyhelminth, and may be selected from the group consisting of trematodes and cestodes, such as *Fasciolopsis, Echinostoma* and *Heterophyes* species, *Clonorchis sinensis, Opisthorchis viverrini, Opisthorchis felineus, Fasciola hepatica, Schistosoma* species, *Diphyllobothrium* species, *Taenia saginata, Taenia solium* and *Hymenolepsis nana*.

In other embodiments, the helminthic parasite is selected from the group consisting of filarial parasites and lung flukes.

In preferred embodiments, the helminthic parasites are selected from the group consisting of *Trichuris muris, Trichinella spiralis, Nippostrongylus prasiliensis, Heligmonsomoides polygyrus, Hymenolepsis nanan, Angiostrongylus* species, *Trichuris suis, Ascaris suum, Trichuris vulpis, Toxocara* species, *Gnathostoma* species, *Ancylostoma* species, *Anisakis* species and *Pseudoterranova* species.

It is preferred that the preparatory animals for parasite production and preparation are rasied in specific pathogen-free (SPF) (e.g., specific human pathogen free) environment.

### DISEASES TREATABLE

Examples of treatable diseases, according to the disclosure, include, but are not limited to, Th1 related and Th2 related diseases, including Th1 or Th2 related cancers.

### Th1-related Diseases

The Th1-related diseases comprise the following groups of diseases: infectious diseases, autoimmune diseases, delayed type hypersensitivity diseases and cancer.

The group of infectious diseases includes diseases caused by parasites and viruses, such as HIV.

The group of autoimmune diseases include encephalomyelopathic, demyelinating and other autoimmune diseases.

Examples of encephalomyelopathic diseases include, but are not limited to, multiple sclerosis (MS); disseminated sclerosis; focal sclerosis; insular sclerosis; tabes dorsalis (posterior sclerosis); acute and chronic experimental allergic (or autoimmune) encephalomyelitis (EAE), which is an animal model of MS; Guillain-Barr syndrome; experimental allergic neuritis (an animal model of Guillain-Barr syndrome); acute disseminated encephalomyelitis; myalgic encephalomyelitis (benign and epidemic); viral encephalomyelitis; granulomatous encephalomyelitis; etc. Also included are animal diseases, such as but not limited to canine distemper; feline distemper; equine encephalomyelitis (eastern, Venezuelan, and western); avian encephalomyelitis; porcine encephalomyelitis; bovine encephalomyelitis; mouse encephalomyelitis; etc.

Examples of demyelinating diseases include, but are not limited to, multiple sclerosis (MS), disseminated sclerosis (DS), acute disseminated encephalomyelitis, progressive multifocal leukoencephalopati (PML), and subacute sclerotic panencephalitis (SSPE).

Examples of other auto-immune diseases include multiple sclerosis (MS), polyartheritis nodosaasthma, hypersensitivity pneumonitis, interstitial lung disease, sarcoidosis, idiopathic pulmonary fibrosis, interstitial lung disease associated with Crohn's disease or ulcerative colitis or Whipple's disease, interstitial lung disease associated with Wegeners granulomatosis or hypersensitivity vasculitis,
vasculitis syndromes, Hennoch-Schonleins purpura, Goodpastures syndrome, Wegeners granulomatosis,
renal diseases such as antibody mediated glomerulopathia as in acute glomerulonephritis, nephritis associated with systemic lupus erythematosus (SLE), nephritis associated with other systemic diseases such as Wegeners granulomatosis and Goodpastures syndrome and mixed connective tissue disease, chronic interstitial nephritis, chronic glomerulonephritis,
gastrointestinal diseases (e.g. IBD), such as Crohn's disease, ulcerative colitis, coeliac disease, Whipple's disease, collagenous colitis, eosinophillic colitis, lymphocytic colitis,
hepatobilliary diseases such as auto-immune hepatitis, alcohol induced hepatitis, periportal fibrosis, primary billiary cirrhosis, sclerosing colangitis,
skin diseases such as psoriasis, atopic dermatitis, eczema, allergic skin disease, progressive systemic sclerosis (scleroderma), exfoliating dermatitis, pemphigus vulgaris,
joint diseases such as rheumatoid arthritis (RA), ankylosing spondylitis, arthritis associated with psoriasis or inflammatory bowel disease,
muscoloskelletal diseases such as myastenia gravis (MG), polymyositis,
endocrine diseases such as insulin dependent diabetes mellitus (IDDM), auto-immune thyroiditis (Hashimoto), thyreotoxicosis, hyperthyroidism (Graves disease),
diseases of the hematopoetic system such as auto-immune anaemia, auto-immune thrombocytopenia, auto-immune aplastic anemia.
cardiovascular diseases such as cardiomyopathia, vasculitis, cardiovascular disease associated with systemic diseases as systemic lupus erythematosus, polyarthritis nodosa, rheumatoid arthritis, scleroderma, sarcoidosis.

A noticeable characteristic of autoimmune diseases is their familial clustering and association with the expression of particular genes, in particular genes of class I and class II major histocompatibility complex (MHC). For example, a large proportion of MS patients have the HLA-DR2 haplotype (Beall S S, Concannon P, Charmley P, et al., J. Neuroimmunol.,v 21, p 59-66, 1989). Since not all subjects with a susceptible genotype develop the autoimmune disease, it appears that environmental factors also play a major role. For example, MS appears to be more common in subjects who live in temperate climate regions (Kurtzke J F, IN: Multiple Sclerosis, Hallpike J F, Adams C W M, Tourtelotte W W, editors, Williams and Wilkins, Baltimore, Md., 1983, p 49-95). It has long been speculated that the environmental factor of autoimmune diseases may be an infectious agent such as a virus. The etilogy of several human and animal diseases can be attributed to viral infection. For example, Theiler's murine encephalomyelitis is a demyelinating disease with clinical and pathological signs similar to EAE. Although antibodies are involved in some effector responses of autoimmune diseases, the triggering event in most cases begins with the activation of CD4 T-cells that are required for B cell maturation and clonal expansion.

The group of delayed type hypersensitivity includes contact hypersensitivity to low molecular weight substances.

### A. Inflammatory bowel diseases (CD and UC):

Epidemiological data suggest a genetic susceptibility to the development of Crohn's disease (CD) and ulcerative colitis (UC). The incidence of CD in industrialized societies has increased from the 1950s until the mid 1980s, and now is from 1 to 8 per 100,000 persons per year. This suggests that unknown changes in our environment have effected the frequency of CD.

While the cause of IBD remains undetermined, it is presumed to result from dysregulation of the intestinal mucosal immune system. Inflammatory cells in the mucosa normally protect us from luminal contents. This highly effective chronic inflammation is tightly controlled to limit tissue injury. IBD may result from inappropriately vigorous immune responses to luminal factors. CD appears to be an overly vigorous Th1-type inflammation that produces IFN-γ and TNFα. The nature of UC is less well defined.

Ulcerative colitis (UC) and Crohn's disease (CD) are disorders of complex derivation caused by the interplay of poorly defined environmental exposures and, at least in some instances, the inheritance of susceptibility genes. Often cited as evidence for genetic predisposition for IBD is the higher than expected occurrence of IBD in family members of patients with this condition and the high prevalence of the disease in Jewish populations of Western countries (Roth MP, Petersen GM, McElree C et al. Geographic origins of Jewish patients with inflammatory bowel disease. Gastroenterology 1989;97(4):900-4). Yet, IBD is much less prevalent in the Jewish population of Israel (Fireman Z, Grossman A, Lilos P et al. Epidemiology of Crohn's disease in the Jewish population of central Israel 1970-1980. Am J Gastro 1989;84(3):255-8) with similar ethnic origin (Grossman A, Fireman Z, Lilos P et al. Epidemiology of ulcerative colitis in the Jewish population of central Israel 1970-1980. Hepato-Gastroenterology 1989;36(4):193-7). Twin studies provide evidence of genetic predisposition for at least CD (Halfvarson J, Bodin L, Tysk C et al. Inflammatory bowel disease in a Swedish twin cohort: a long-term follow-up of concordance and clinical characteristics. Gastroenterology 2003;124(7):1767-73.). A genetic defect in CARD15/NOD2, an intracellular protein that senses the bacterial product muramyl dipeptide (Inohara M, Ogura Y, Fontalba A et al. Host recognition of bacterial muramyl dipeptide mediated through NOD2: implications for Crohn's disease. J Biol Chem 2003; Girardin SE, Boneca IG, Viala J et al. Nod2 is a general sensor of peptidoglycan through muramyl dipeptide (MDP) detection. J Biol Chem 2003), leaves some people more susceptible to CD. Various other genetic alterations are proposed as IBD risk factors. Yet, genetic predispositions do not explain the rapidly increasing incidence of disease.

There are several animal models of chronic intestinal inflammation, for example, as reported by Elliott et al. (Elliott et al., 1998, Inflammatory Bowel Disease and Celiac Disease. In: The Autoimmune Diseases, Third ed., N.R. Rose and I.R. MacKay, eds. Academic Press, San Diego, CA). An important advance is the recent discovery that some mice with genetically engineered gene deletions can develop chronic bowel inflammation similar to IBD, see Elson et al., 1995, Gastroenterology 109:1344. These include mutant mice bearing targeted deletions for IL-2, IL-10, MHC class II or TCR genes among others. Using some of the models, Berg et al., 1996, J. of Clin. Investigation 98:1010, Ludviksson et al., 1997, J. of Immunol. 158:104, and Mombaerts et al., 1993, Cell 75:274 have shown that a dysregulated immune system itself can mediate intestinal injury. The mucosal inflammation of several of these models generates large amounts of IFN-γ and TNF-α suggesting that excess production of Th1-type cytokines is one common mechanism underlying the pathogenesis of disease. Also, blocking Th1 circuitry prevents the inflammation. CD is a Th1 response. Thus, these models may have direct implications regarding the immunopathology of this human disease process.

### B. Rheumatoid arthritis (RA):

RA is a chronic disease featuring persistent inflammatory synovitis, usually involving peripheral joints in a symmetric distribution. This inflammation can lead to bone erosions, cartilage damage and joint destruction. It is an affliction of about 1% of the population. The prevalence increases with age, and women are affected more frequently than men. The propagation of RA is an immunologically mediated event driven by CD4+ Th1 cells.

### C. Insulin-dependent, Juvenile Diabetes Mellitus (DM)(type 1):

Type 1 DM is a disease that usually begins during early adulthood and that results from the inability to produce insulin in response to increasing blood sugar. This persistent high blood sugar and inability to properly metabolize glucose causes metabolic disturbances that eventually damage the eyes, kidneys, heart and other organs. Taking insulin parenterally can partially control these metabolic problems. Type 1 DM results from an autoimmune attack on the pancreatic beta cells, which are the source of insulin. Activated macrophages and cytotoxic T cells surround and destroy the pancreatic beta cells. Genetic susceptibility and poorly defined environmental events trigger the disease process.

### D. Lupus Erythematosus (LE):

LE is a systemic autoimmune disease that is most frequent in women of early to middle adulthood. The tissue damage is caused by autoantibodies and hyper-reactive regulatory T cells. The abnormal immune response allows sustained production of pathogenic autoantibodies and immune complexes. This leads to damage of the musculoskeletal, cutaneous, hematologic, renal and other systems. The abnormal immune response probably depends upon the interaction of multiple hereditary and environmental factors.

### E. Sarcoidosis:

Sarcoidosis is a chronic granulomatous disease of the lungs and other organs of unknown cause. Most patients present between the ages of 20-40. The most frequent symptom is shortness of breath. The disease results from an exaggerated Th1-type, cellular immune response, probably to a limited number of antigens. Sarcoidosis develops throughout the world, and afflicts all races. However, there is remarkable diversity of the prevalence of sarcoidosis among certain ethnic and racial groups. For instance, the disease is rare in Poland, Southeast Asia and India.

### F. Multiple Sclerosis (MS):

MS is a chronic relapsing, multifocal inflammatory disorder of the central nervous system that leads to focal demyelination and scarring of the brain. It is a frequent disease affecting about 350,000 Americans and which begins during early to middle adulthood. MS is an autoimmune disease mediated at least in part by Th1 cells. The lesions of MS resemble those induced by delayed hypersensitivity responses that contain activated T cells and macrophages. It is a disease of temperate climates, increasing in prevalence with distance from the equator.

### Th2-related Diseases

The Th2-related diseases comprise the following groups of diseases: Allergic diseases and cancer.

It is well known that genetically predisposed individuals become sensitised (allergic) to antigens originating from a variety of environmental sources, of which the individuals are exposed. The allergic reaction occurs when a previously sensitised individual is re-exposed to the same or a homologous allergen. Allergic responses range from hay fever, rhinoconductivitis, rhinitis and asthma to systemic anaphylaxis and death in response to e.g. bee or hornet sting or insect bite. The reaction is immediate and can be caused by a variety of atopic allergens such as compounds originating from grasses, trees, weeds, insects, (house dust) mites, food, drugs, chemicals and perfumes.

The group of allergic diseases includes hay fever, rhinoconjunctivitis, rhinitis and asthma.

The most common allergens, to which allergic reactions occur, include inhalation allergens originating i.e. from trees, grasses, herbs, fungi, house dust mites, storage mites, cockroaches and animal hair, feathers, and dandruff. Important pollen allergens from trees, grasses and herbs are such originating from the taxonomic orders of Fagales, Oleales and Pinales including i.e. birch (Betula), alder (Alnus), hazel (Corylus), hornbeam (Carpinus) and olive (Olea), the order of Poales including i.a. grasses of the genera Lolium, Phleum, Poa, Cynodon, Dactylis and Secale, the orders of Asterales and Urticales including i.a. herbs of the genera Ambrosia and Artemisia. Important inhalation allergens from fungi are i.a. such originating from the genera Alternaria and Cladosporium. Other important inhalation allergens are those from house dust mites of the genus Dermatophagoides, storage mites from the genus Lepidoglyphys destructor, those from cockroaches and those from mammals such as cat, dog, horse, cow, and bird. Also, allergic reactions towards stinging or biting insects such as those from the taxonomic order of Hymenoptera including bees, wasps, and ants are commonly observed. Specific allergen components are known to the person skilled in the art and include e.g. Bet v 1 (B. verrucosa, birch), Aln g 1 (Alnus glutinosa, alder), Cor a 1 (Corylus avelana, hazel) and Car b 1 (Carpinus betulus, hornbeam) of the Fagales order. Others are Cry j 1 (Pinales), Amb a 1 and 2, Art v 1 (Asterales), Par j 1 (Urticales), Ole e 1 (Oleales), Ave e 1, Cyn d 1, Dac g 1, Fes p 1, Hol 11, Lol p 1 and 5, Pas n 1, Phi p 1 and 5, Poa p 1, 2 and 5, Sec c 1 and 5, and Sor h 1 (various grass pollens), Alt a 1 and Cha h 1 (fungi), Der f 1 and 2, Der p 1 and 2 (house dust mites, D. farinae and D. pteronyssinus, respectively), Lep d 1, Bla g 1 and 2, Per a 1 (cockroaches, Blatella germanica and Periplaneta americana, respectively), Fel d 1 (cat), Can f 1 (dog), Equ c 1, 2 and 3 (horse), Apis m 1 and 2 (honeybee), Ves g 1, 2 and 5, Pol a 1, 2 and 5 (all wasps) and Sol i 1, 2, 3 and 4 (fire ant), to mention the most common.

### A. Asthma

In many people, asthma appears to be an allergic reaction to substances commonly breathed in through the air, such as animal dander, pollen, or dust mite and cockroach waste products. The catch-all name for these substances, allergens, refers to anything that provokes an allergic reaction. Some people have a genetic predisposition to react to certain allergens. In 1998, an estimated 17 million Americans, or 6.4 percent of the population, had asthma. Children account for 4.8 million of Americans with asthma. Available experimental and clinical information from both animal models as well as clinical information on the pathology of human asthma indicates Th2 cells in the pathology of this disease.

### Th1- and Th2-related Cancers

In general, cancer cells may raise an immune response to antigens specifically expressed by the cancer cell. Since both Th1 and Th2 immune responses may drive strong inflammatory responses leading to cytotoxic eradication of tissue, modulation of Th1 or Th2 immune responses may be used in the treatment of cancer.

Cancers, which can be treated with the composition according to the disclosure can be histogenetically classified as malignant epithelial tumours, including carcinomas and adenocarcinomas, and as malignant non-epithelial tumours, including liposarcomas, fibrosarcomas, chondrosarcomas, osteosarcomas, leiomyosarcomas, rhabdomyosarcomas, gliomas, neuroblastomas, medulloblastomas, malignant melanoma, malignant meningioma, various leukemias, various myeloproliferative disorders, various lymphomas (Hodgkin's lymphoma and non-Hodgkin lymphoma), haemangiosarcoma, Kaposi's sarcoma, lymphangiosarcoma, malignant teratoma, dysgerminoma, seminoma, and choricarcinoma. Carcinomas and adenocarcinomas are the most abundant (accounting for approximately 90% of deaths from cancer) and are therefore interesting target diseases to treat/prevent according to the disclosure. The most important carcinomas and adenocarcinomas are those of the airways (especially of bronchial origin), of the breast, of the colorectum and of the stomach. However, also carcinomas and adenocarcinomas of the prostate, the ovary, of the lymphoid tissue and bone marrow, of the uterus, of the pancreas, of the esophagus, the urinary bladder, and the kidney cause a significant number of deaths and are therefore of interest.

The group of cancer diseases further includes Sezary's syndrome, cutaneous T-cell lymphoma, hepatic carcinoma and lung cancer.

### REGULATORY T CELLS IN TH1 OR TH2 RELATED DISEASES

Regulatory T cells can induce peripheral tolerance and limit mucosal reactivity (McGuirk P, Mills KH. Pathogen-specific regulatory T cells provoke a shift in the Th1/Th2 paradigm in immunity to infectious diseases. Trends Immunol 2002;23(9):450-5). In various animal models, several regulatory T cell phenotypes have been reported. Regulatory T cells express various markers and have been indicated to involve in different diseases (See, e.g., Field, A. C., L. Caccavelli, M. F. Bloch, and B. Bellon. 2003. Regulatory CD8+ T cells control neonatal tolerance to a Th2-mediated autoimmunity. Journal of Immunology. 170:2508-2515; von Herrath MG, Harrison LC. Antigen-induced regulatory T cells in autoimmunity. Nat Rev Immunol. 2003 Mar;3(3):223-32; Francois Bach J. Regulatory T cells under scrutiny. Nat Rev Immunol. 2003 Mar;3(3):189-98; Curotto de Lafaille MA, Lafaille JJ. CD4(+) regulatory T cells in autoimmunity and allergy. Curr Opin Immunol. 2002 Dec; 14(6):771-8; McGuirk P, Mills KH. Pathogen-specific regulatory T cells provoke a shift in the Th1/Th2 paradigm in immunity to infectious diseases. Trends Immunol. 2002 Sep;23(9):450-5; Tung KS, Agersborg SS, Alard P, Garza KM, Lou YH. Regulatory T-cell, endogenous antigen and neonatal environment in the prevention and induction of autoimmune disease. Immunol Rev. 2001 Aug; 182:135-48; Read S, Powrie F. CD4(+) regulatory T cells. Curr Opin Immunol. 2001 Dec;13(6):644-9; Yssel H, Lecart S, Pene J. Regulatory T cells and allergic asthma. Microbes Infect. 2001 Sep;3(11):899-904). In some systems, they are distinguished through differential expression of surface molecules like CD25 (Shevach, E. M. 2002. CD4+ CD25+ suppressor T cells: more questions than answers. Nature Reviews.Immunology. 2:389-400), CD45RB (Annacker, O. and F. Powrie. 2002. Homeostasis of intestinal immune regulation. Microbes & Infection. 4:567-574), and CTLA-4 (Read, S., V. Malmstrom, and F. Powrie. 2000. Cytotoxic T lymphocyte-associated antigen 4 plays an essential role in the function of CD25(+)CD4(+) regulatory cells that control intestinal inflammation. J.Exp.Med. 192:295-302). This pattern of cell surface protein expression suggests that they may be in a primed effector or memory state. These regulatory cells may mediate some of their affects through production of IL 10 and TGFβ. Described is an anergic regulatory T cell (Tr1) that produces high levels of IL10 and TFGβ. Another cell called Th3 suppresses induction of experimental autoimmune encephalomyelitis primarily through production of TGFβ. Still others are not dependent on soluble IL10 or TGFβ, but instead express on their surface latency-associated peptide, which is the amino-terminal domain of the TGFβ precursor peptide(Oida T, Zhang X, Goto M et al. CD4+CD25- T cells that express latency-associated peptide on the surface suppress CD4+CD45RBhigh-induced colitis by a TGF-beta-dependent mechanism. J Immunol 2003;170(5):2516-22). Internal cell markers have also been reported for regulatory T cells (Schubert LA, Jeffery E, Zhang Y, Ramsdell F, Ziegler SF, Scurfin (FOXP3) acts as a repressor of transcription and regulates T cell activation. J Biol Chem. 2001 Oct 5;276(40):37672-9).

*Rag* mice reconstituted with CD4+, CD45^{high} T cells can develop severe colitis, which can be prevented by co-transfer of CD4+, CD45^{low} T cells (Annacker O, Powrie F. Homeostasis of intestinal immune regulation. Microbes & Infection 2002;4(5):567-74). TGFβ and IL10 are required for protection suggesting a role for these cytokines in the regulatory process.

### HELMINTHIC PARASITE PREPARATIONS

Helminths are parasitic animals (worms), which, depending on species, live in locations like the intestinal lumen, blood stream or muscles of the host. These organisms colonize more than 1/3 of the world population. Helminth colonization is most common in children living in warm climates and subject to poor sanitation. The infective forms of these organisms are spread through contact with contaminated soil, food or water. Before the 1940s, many children and adults in the United States carried helminths. Worm carriage was particularly common in rural areas of the South and in indigent populations of major cities (Elliott DE, Urban JF, Jr., Argo CK et al. Does the failure to acquire helminthic parasites predispose to Crohn's disease? FASEB J 2000; 14(12): 1848-55). In the United States and Europe, helminthic colonization has steadily declined. They are found in recent immigrants from less developed countries (Salas SD, Heifetz R, Barrett-Connor E. Intestinal parasites in Central American immigrants in the United States. Arch Intern Med 1990; 150(7):1514-6) and in economically disadvantaged populations living in underserved regions of the United States like some Indian reservation (Healy GR, Gleason NN, Bokat R et al. Prevalence of ascariasis and amebiasis in Cherokee Indian school children. Public Health Rep 1969;84(10):907-14). The prevalence of helminths is highest in warm climates and in populations subject to crowding, poor sanitation and impure food supply. Inflammatory bowel disease (IBD), rheumatoid arthritis and autoimmune diseases are rare in these same regions.

Nematodes that frequently inhabit the human gut are Ascaris lumbricoides, Enterobius vermicularis (pin worm), Trichuris trichiura (whipworm), Ancylostoma duodenale and Necator americanus (hookworms), and Strongyloides stercoralis. Trichinella spiralis infests the small intestine briefly.

The platyhelminths include the trematodes and cestodes. The most common adult trematodes that reside in the human intestines are *Fasciolopsis, Echinostoma* and *Heterophyes* species. Those that live in the biliary system include *Clonorchis sinensis, Opisthorchis viverrini* and *felineus,* and *Fasciola hepatica. Schistosoma* dwell in the venous system, but several species chronically affect the gut by the passage of eggs through the intestinal wall. Adult cestodes commonly infecting humans are *Diphyllobothrium* species (fish tapeworm), *Taenia saginata* (beef tapeworm), *Taenia solium* (pork tapeworm) and *Hymenolepsis nana* (dwarf tapeworm).

The host acquires various helminthic species through contact with soil, food or water contaminated with the infective form of the parasite. Children most frequently harbor helminthic infections because of their close contact with soil and suboptimal hygienic practices. Helminths incite an intestinal Th2 response, which can cause worm expulsion or limit the magnitude of infection. Most children living in non-industrialized countries have these parasites. Many helminthic species survive for years within the gut, biliary tree or mesenteric veins making thousands of eggs daily. Thus, beginning in childhood, these worms and/or their ova release molecules that bathe the intestinal mucosal surface for years inciting Th2-type inflammation.

### A. Viable Organism Vaccines:

Viable helminthic parasite organisms may provide the most profound mucosal conditioning because of their relative longevity as compared to component vaccines. Viable organisms can be administered in either egg, larval, cercarial, or encysted larval forms depending on the helminth. Helminths that can colonize humans and a preparatory animal may be utilized.

The preparatory animal may need manipulation to allow high patency by the helminthic. Such manipulation can include treatment with agents that are immunosuppressive like glucocorticoids or azathioprine; agents that impede Th2 effects like anti-histamines, anti-cytokines, or recombinant cytokines; and agents that influence intestinal motility like anticholinergics or opiates. The animal's diet will be altered to reduce coarse fiber content. The animal may be a rat, pig, hamster, bird or other preparatory animal.

Preparatory animals are raised in specific pathogen-free (SPF) (e.g. specific human pathogen free)environments according to methods known in the art. They are tested to ensure absence of human bacterial, mycobacterial, and viral pathogens. Methods for raising animals in SPF environment and advantage thereof is described in the art, e.g., by M. Michael Swindle (J. Invest. Surg., 9:267-271, 1996). Swindle also lists several potential human viral and bacterial pathogens of concern. Accordingly, parasite preparations derived from animals raised in a specific human pathogen free environment would not contain these human pathogens, and as such are patentably distinct from parasite preparations prepared from animals raised in any SPF environment

Eggs: Some intestinal helminths are acquired by ingestion of viable eggs. Helminths are maintained in SPF preparatory animals, for example, SPF pigs. To harvest eggs, the animals are given a special diet low in coarse fiber. Animals are given an oral purgative to induce defecation. Stool is collected and enzymatically digested to free eggs. Eggs are isolated from liquefied stool by flotation on density gradients, screen filtration, Visser filtration, or centrifugal elutriation. Preservation of eggs varies with the helminth used. Eggs from helminths that are resistant to dessication are dried, compounded with inert material, incorporated into an enteric capsule, and refrigerated. Eggs from helminths that are susceptible to dessication are preserved by refrigeration in liquid medium or by adding cryoprotectant and freezing in liquid nitrogen. Viable eggs are washed, mixed with chilled lactose-free pudding or other vehicle at the location for delivery. Eggs stored in glycerol-based cryoprotectant may not require washing. Eggs from each lot are tested for hatching rate to determine effective dosing. Eggs from each lot are tested for absence of bacterial and viral pathogens.

Larvae: Some helminths (i.e. hookworms) require a soil maturation phase before they can colonize humans. Eggs from these agents will be incubated under optimal conditions to mature the embryo, or hatch the egg and provide larval forms. Patients can be inoculated by subcutaneous injection or oral ingestion of viable larvae.

**Cercariae:** Some helminths have complex life cycles that utilize an intermediate host. The intermediate host sheds the form able to colonize humans. Cercariae are the form for trematode helminths (i.e. flukes) shed by intermediate hosts like snails. Cercariae are isolated from colonized snails grown in SPF conditions. Cercariae are washed. These may be preserved by adding cryoprotectant and freezing in liquid nitrogen. Thawed or fresh cercariae are washed and injected subcutaneously to inoculate patients. Samples from each lot are tested for absence of pathogens and to determine effective dose.

**Encysted Larvae:** Some helminths (e.g. tapeworms) form encysted larvae or cysticerci in intermediate hosts. It is the encysted larval form that initiates human colonization. Encysted larva are removed from intermediate hosts, for example, cattle or fish or plants grown in SPF conditions. Cysts are washed free of remaining host tissue. Cysts may be preserved by adding cryoprotectant and freezing in liquid nitrogen. Cysts are thawed or used fresh, washed, mixed with chilled lactose-free pudding or other vehicle at the location for delivery and fed to individuals. Samples from each lot are tested for absence of pathogens and to determine effective dose.

### B. Non-viable Component Vaccines:

Non-viable components of helminthic parasites provide sufficient Th2 conditioning of the immune response to prevent Th1-mediated pathology. Non-viable components are derived from eggs, larvae or adult worms.

Non-viable, intact schistosome eggs produce a strong Th2 response. Eggs are isolated from livers of preparatory animals (i.e. hamsters) grown under SPF conditions. Eggs are isolated by a method modified from that originally described by Coker and Lichtenberg, 1956, Proceedings of the Soc. For Exp. Biol. & Med. 92:780. The modifications consist of using phosphate buffered saline with glucose instead of 1.7% saline for incubation and washing steps along with decreasing the autolytic digestion time. These changes promote isolation of viable eggs. The method is as follows.

Infect golden hamsters with 1000 to 1500 cercariae. Allow the infection to mature (6 to 7 weeks). Remove the livers from the animals and place in 600 mOsm sterile phosphate buffered saline containing 5% glucose, 100 U/ml penicillin and 100 mg/ml streptomycin. The livers are allowed to autodigest for 24 hours at room temperature. Pulse homogenize the livers at low speed for 3 minutes in a cold Waring blender. Incubate the homogenate with collagenase (2 mg/ml) and trypsin (2 mg/ml) at 32°C for one hour. Filter the homogenate through 50 and 80-100 mesh sieves to remove clumps of tissue and debris. Recover the eggs from the filtrate by passing over a 325 mesh sieve. The eggs will not pass through the screen. Flush the eggs off of the screen and into a 50ml polypropylene centrifuge tube. Wash the eggs by repeated low speed (400 x g) centrifugation in sterile phosphate buffered saline with 5% glucose. The eggs must be free of any collagenous debris. Count an aliquot of eggs in a 1ml Sedwick chamber to determine total egg number.

Isolated eggs are suspended in saline and flash frozen in liquid nitrogen without cryoprotectant. This kills the egg. Thawed eggs are injected subcutaneously, intramuscularly or intravenously, or at sites of Th1 inflammation to elicit strong Th2 responses. Eggs from other helminths may also be utilized.

Component vaccines may also be used that employ proteins, lipids, or carbohydrates isolated from parasite eggs. An example is schistosome soluble egg antigens (SEA). The method for preparing schistosome egg antigen has been previously described by Boros and Warren, 1970, J. of Experimental Med. 132:488 and is briefly given below.

Washed eggs are resuspended at 50,000 eggs/ml of phosphate buffered saline. This is transferred to a glass tissue homogenizer. The eggs are homogenized on ice. To insure that all shells are broken and miracidia are disrupted, an aliquot (5ml) is removed for microscopic inspection. Transfer the homogenate to ultracentrifuge tubes. Centrifuge at 100,000 x g for 2 hours at 4°C. Recover the aqueous fraction (SEA) and determine the protein content. Store the SEA in small aliquots at -70°C. This method may require modification to isolate the parasite egg products that most strongly promote Th2 conditioning, i.e., to achieve an optimal effective concentration, (100µg SEA or 10,000 ova/animal). Eggs or soluble egg components are used to initiate Th2 responses or to boost Th2 responses previously initiated by colonization with viable helminths. Eggs or egg components are tested to confirm the absence of pathogens and endotoxin.

Component vaccines can also be developed from larvae and adult worms of helminthic parasites. Larvae or worms are isolated from preparatory animals grown in SPF conditions. Vaccines that employ non-viable intact organisms or proteins, lipids, or carbohydrates isolated from the helminth are prepared and utilized in a manner similar to that previously described for helminth eggs.

Also encompassed in the present disclosure are vaccines containing fractions or subfractions of the helminthic parasites, as well as vaccines containing isolated proteins, polynucleotides, carbohydrates, lipids, etc. derived from helminthic parasites according to known methods in the art, e.g., as described in Williams et al., 1994, Leukocytes of patients with Schistosoma mansoni respond with a Th2 pattern of a cytokine production to mitogen or egg antigens but with a Th0 pattern to worm antigens. J. Infect. Dis. 170:946; Xu-Amano et al., 1993, Helper T cell subsets for immunoglobulin A responses: oral immunization with tetanus toxoid and cholera toxin as adjuvant selectively induces Th2 cells in mucosa associated tissues. J. Exp. Med. 178:1309; Ausiello et al., 2000, Cell mediated immunity and antibody responses to Bordetella pertussis antigens in children with a history of pertussis infection and in recipients of an acellular pertussis vaccine. J. infect. Dis., 181:1989.

For example, fractions or subtractions may be prepared according to w method described in Thaumaturgo et al. (2001, Preliminary Analysis of Sm14 in Distinct Fractions of Schistosoma mansoni Adult Worm Extract, Mem. Inst. Oswaldo Cruz vol.96 suppl. Vol. 96, Suppl.: 79-83 ). In this method, S. mansoni parasites were obtained by retrograde perfusion with heparinized saline (0.85% NaCl solution), 45 days post-infection (Pellegrino & Siqueira 1956, Técnica de perfusão para colheita de Schistosoma mansoni em cobaias experimentalmente infectadas. Rev Bras Mal D Trop 8: 589-597), and used for preparing the different extracts.

--Preparation of adult-worm-derived extract (SE) - Parasites were rinsed in PBS. Living worms were then allowed to stand at room temperature (28°C to 30°C) in fresh PBS (phosphate buffered saline) for 2-3 h, and frozen at -20°C. After thawing, PBS suspensions of the worms (1g worms/10 ml PBS) were filtered through a wire mesh, and centrifuged at 10,000 g for 1 h at 4°C (Tendler & Scapin 1981, Schistosoma mansoni antigenic extracts obtained by different extraction procedures. Mem Inst Oswaldo Cruz 76: 103-109; Tendler et al. 1982, Immunogene and protective activity of an extract of Schistosoma mansoni. Mem Inst Oswaldo Cruz 77: 275-283). The protein content of SE was assessed by Lowry's method (Lowry et al. 1951), and stock batches containing 1 mg/ml were stored at -20°C. Male and female extracts were prepared according to the same methodology, with parasites separated immediately after perfusion.

Preparation of a rapidly released "fraction" of adult-worm-derived extract (SEi) - SEi preparation corresponded to the initial step of SE: after perfusion as described for SE, live worms were incubated for 2-3 h at room temperature (28°C to 30°C), in PBS. Thereafter, adult worms were filtered from incubation solution (SEi) in a wire mesh.

Preparation of SE2 - Adult worms remained from SE initial preparation, were re-stored frozen (-20°C) in PBS for one week (1g worms/10 ml PBS). After thawing, PBS suspensions were filtered through a wire mesh, and centrifuged at 10,000 g for 1 h at 4°C.

Parasite antigens can also be extracted as described in figure 1 of Thaumaturgo et al. (supra) (reproduced as Figure 23). Alternatively, parasite antigens may be prepared as described in Deelder et al. (1980. Applicability of different antigen preparations in the enzyme-linked immunosorbent assay for schistosomiasis mansoni. Am. J. Trop. Med. Hyg. 29:401-410). For example, antigens may be prepared from SWAP prepared as soluble supernatant fluids from buffered saline homogenates of the respective life-cycle stage (Goes et al. 1989, Production and characterization of human monoclonal antibodies against Schistosoma mansoni. Parasite Immunol 11: 695-711). SWAP was fractionated by anion-exchange chromatography on FPLC (fast protein liquid chromatography), as previously described (Hirsch & Goes 1996, Characterization of fractionated Schistosoma mansoni soluble adult worm antigens that elicit human cell proliferation and granuloma formation in vitro. Parasitology 112: 529-535). Briefly, proteins were eluted with 20 mM Tris-HCl, pH 9.6, in a multistep increasing gradient up to 1 M NaCl, interrupted by hold-gradient intervals at 0, 100, 280, 450, 600 and 750 mM. Flow-through fractions were concentrated by lyophilization. The concentrated material was dialyzed against 0.15 M phosphate-buffered saline (PBS), pH 7.4, sterilized by filtration and stored at -70°C. The protein content was measured according to Bradford microassay (Bradford 1976, A rapid and sensitive method for the quantification of microgram quantities of protein utilizing the principle of protein-dye binding. Analyt Biochem 72: 248-254 ). Analysis of the six fractions, separated by 10% SDS-PAGE (sodium dodecylsulfate polyacrylamide gel electrophoresis) under reducing conditions (Laemmli 1970, Cleavage of structural proteins during the assembly of the head of bacteriophage T4. Nature 227: 680-685 ), showed multiple protein bands, fraction III containing high (97 and 160 KDa), intermediate (52 and 56 KDa) and low (28 and 36 KDa) proteins. This fraction was called PIII and was used in different immunological assays.

Lipids may also be prepared as described in the art, for example, in van der Kleij et al. (1999, Recognition of Schistosome Glycolipids by Immunoglobulin E: Possible Role in Immunity Infection and Immunity, 67: 5946-5950 ).

In one embodiment, lipids of *S. mansoni* adult worms (12 g [wet weight]) and eggs (1.6 g [wet weight]) were extracted by the method described by Bligh and Dyer (1959. A rapid method of total lipid extraction and purification. Can. J. Biochem. Physiol. 37:911-917). The organic phase was dried by rotary evaporation, dissolved in 10 ml of chloroform, and applied to a 20-ml column of the anion exchanger TEAE-cellulose (Serva, Heidelberg, Germany) that was converted to the hydroxyl form. Lipids were eluted as described by Rouser et al. (1969. Diethylaminoethyl and triethylaminoethyl cellulose column chromatographic procedures for phospholipids, glycolipids, and pigments. Methods Enzymol. 14:272-317). According to this protocol, the fractions contain the following lipids: fraction 1, cholesterol, glycerides, and other neutral lipids; fraction 2, cerebrosides, glycerol diglycerides, phosphatidylcholine, and sphingomyelin; fraction 3, ceramidepolyhexosides; fraction 4, inorganic substances; fraction 5, phosphatidylethanolamine and free fatty acids; fraction 6, phosphatidylserine; fraction 7, none (washing step); and fraction 8, phosphatidic acid, cardiolipin, phosphatidylglycerol, phosphatidylinositol, and other acidic lipids. The presence of glycolipids in fractions 2 and 3 was confirmed by orcinol staining of the lipid fractions on HPTLC plates (1956. Quantitative estimation of cerebrosides in nervous tissue. J. Neurochem. 1:42-53).

### C. Maintenance of Helminth Organisms:

Helminths are cycled through intermediate and preparatory animals grown in SPF conditions. Samples of helminth populations are tested to ensure phenotypic stability such as colonization rates, fecundity, and susceptibility to anti-helminthics.

### DOSAGE, ADMINISTRATION AND PHARMACEUTICAL FORMULATIONS

Individuals in need of treatment receive the infected form of the parasite (egg, cercariae or larvae) orally or parenterally depending upon the natural life cycle of the parasite selected. Alternatively, soluble worm or egg extracts can be given orally or parenterally to induce TH2 responses.

With regard to intestinal and liver helminths and schistosomes, they begin producing ova that appear in the stool about 30-60 days after inoculation. Quantifying the eggs in the stool proves satisfactory for assessing adequacy and intensity of infection. Aliquots of stool are processed by sucrose floatation to determine the total number of eggs in each specimen. Flotation over sucrose solution is a method frequently used to isolate eggs from stool for accurate counting as reported by Koontz and Weinstock, 1996, Gastroenterology Clinics of N. America 25:435.

The helminthic parasite compounds of the disclosure may be formulated for administration in any convenient way, and the disclosure therefore includes pharmaceutical compositions comprising the helminthic parasite compound in accordance with the disclosure adapted for use in humans. Such compositions may be presented for use in conventional manner with the aid of any necessary pharmaceutical carriers or excipients.

The helminthic parasite compound according to the disclosure may be formulated for injection, and therefore, vaccine use, and may be presented in unit dose form in ampules, or multidose containers, if necessary, with an added preservative. The compositions may also take such forms as suspensions, solutions or emulsions of oily or aqueous vehicles and may contain formulatory agents such as suspending, stabilizing, and/or dispersing agents. Alternatively, the helminthic parasite may be in powder form or reconstituted with a suitable vehicle, e.g. sterile-pyrogen-free water, before use.

If desired, such powder formulation may contain an appropriate non-toxic base in order to ensure that the powder is reconstituted with water, the pH of the resulting aqueous formulation being physiologically acceptable.

The helminthic parasite compounds may also be formulated as suppositories, e.g. containing conventional suppository bases such as cocoa butter or other glycerides.

The helminthic parasite compounds may also be formulated to oral dosage with conventional fillers, carriers, and excipients. The amount of parasite administered to the individual in need thereof is an amount sufficient to prevent or treat the autoimmune disease. This amount may vary depending upon the disease being treated or prevented and the helminthic parasite, whether it is being administered intact, or as an egg, larvae, extract or cercariae.

Typically, when the parasites are administered for all autoimmune diseases discussed herein, the amount ranges from about 50 to about 50,000. More particularly, this amount may range from about 500 to about 5,000. When eggs are utilized, about 500 to about 5000 may be utilized to treat the autoimmune diseases disclosed herein. When extracts are administered, about 100 µg to about 10,000 pg are utilized to treat the autoimmune diseases. When larvae and cercariae are administered, the dosages may range from about 500 to about 5,000 in each case.

For prevention or vaccine use, the amounts of the parasites may be 500-5,000.

Dosage of a parasite preparation may be monitored by measuring Th1, Th2 or regulatory cell responses. Alternatively, the dosage may be monitored by evaluating the pathology or symptoms of a particular disease as known in the art or described herein.

### A. Determination of Th1 and Th2 responses

In order to show the efficacy of the present invention, the Th1 and Th2 response may be distinguished. Metawali et al., 1996, J. of Immunol. 157:4546 has shown that in mice, it is possible to distinguish a Th1 from a Th2 response by histologic analysis, and by analysis of cytokine and immunoglobulin profiles. Further, Sandor et al., 1990, J. of Exp. Med. 171:2171 has shown that cell surface expression of Fcg3 and MHC Class II molecules afford discrimination. In this procedure, small bowel and colon are examined histologically to determine the degree of mucosal inflammation, eosinophilia and mastocytosis. The latter cell types are indicative of a Th2 response. Mesenteric lymph nodes (MLN) and spleens can be dissociated into single cell suspensions for *in vitro* culture in microwell plates. Cells (1-2 x 10⁶ /well) in complete RPMI medium are cultured for up to 72 h in the presence or absence of worm antigen or anti-CD3 and then the supernatants are assayed for cytokines and immunoglobulins. IFN-γ, TNFα and IgG2a characterize a Th1 response, whereas IL-4, IL-5, IgE and IgG1 typify a Th2 reaction. Also, serum can be assayed for cytokine and immunoglobulin concentrations. Furthermore, dispersed inflammatory leukocytes are examined by flow cytometry for Fcy3 expression on macrophages (Th1) and MHC Class II expression on B cells (Th2). Controls include serum, MLN and spleens from appropriate age-matched, littermate mice that hosted no parasite.

A similar analysis can differentiate a human Th1 from a Th2 response. One examines inflamed tissue, isolated leukocytes from regions of inflammation and peripheral blood cells. Leukocytes are cultured in vitro alone or in the presence of parasite antigen or mitogens to stimulate cytokine release. IgG2 substitutes for IgG2a.

### B. Determination of regulatory T cell activity

In the present invention regulatory T-cell activity is determined by determining the level of scurfin.

Methods of isolating regulatory T cells from in vitro culture or animals are known in the art, for example, as described in McGuirk P, Mills KH. Pathogen-specific regulatory T cells provoke a shift in the Th1/Th2 paradigm in immunity to infectious diseases. Trends Immunol 2002;23(9):450-5; Field, A. C., L. Caccavelli, M. F. Bloch, and B. Bellon. 2003. Regulatory CD8+ T cells control neonatal tolerance to a Th2-mediated autoimmunity. Journal of Immunology. 170:2508-2515; von Herrath MG, Harrison LC. Antigen-induced regulatory T cells in autoimmunity. Nat Rev Immunol. 2003 Mar;3(3):223-32; Francois Bach J. Regulatory T cells under scrutiny. Nat Rev Immunol. 2003 Mar;3(3):189-98; Curotto de Lafaille MA, Lafaille JJ. CD4(+) regulatory T cells in autoimmunity and allergy. Curr Opin Immunol. 2002 Dec;14(6):771-8; McGuirk P, Mills KH. Pathogen-specific regulatory T cells provoke a shift in the Th1/Th2 paradigm in immunity to infectious diseases. Trends Immunol. 2002 Sep;23(9):450-5; Tung KS, Agersborg SS, Alard P, Garza KM, Lou YH. Regulatory T-cell, endogenous antigen and neonatal environment in the prevention and induction of autoimmune disease. Immunol Rev. 2001 Aug;182:135-48; Read S, Powrie F. CD4(+) regulatory T cells. Curr Opin Immunol. 2001 Dec;13(6):644-9; Yssel H, Lecart S, Pene J. Regulatory T cells and allergic asthma. Microbes Infect. 2001 Sep;3(11):899-904; Shevach, E. M. 2002. CD4+ CD25+ suppressor T cells: more questions than answers. Nature Reviews.Immunology. 2:389-400; Annacker, O. and F. Powrie. 2002. Homeostasis of intestinal immune regulation. Microbes & Infection. 4:567-574; Read, S., V. Malmstrom, and F. Powrie. 2000. Cytotoxic T lymphocyte-associated antigen 4 plays an essential role in the function of CD25(+)CD4(+) regulatory cells that control intestinal inflammation. J.Exp.Med. 192:295-302.

Regulatory T cell response or activity may be measured by an internal marker, a cell surface marker, or a secreted marker as described herein.

Useful internal markers for regulatory T cells include, but are not limited to, transcription factors such as Scurfin, Smad7, Gata3, Tbet (Tbx21).

Useful surface markers for regulatory T cells include, but are not limited to, CD4, CD45RB^{lo}, CD45Rc, Cytplytic T lymphocyte associated antigen 4 (CTLA-4), CD25, CD103, Ox40, 4-1BB, CD62L, α_{Eβ} integrin, latency-associated peptide (LAP) or glucocorticoid induced TNF receptor family related protein (GITR), chemokine receptor CCR5, TI-ST2.

Useful secreted markers for regulatory T cells include, but are not limited to, IL-5, IL-10 and TGFβ.

### C. Examples of Methods for in-Vitro Measuring the Amount of Markers

**Cytokine Detection by Flow Cytometry:** Splenocytes, MLN or intestinal inflammatory cells in RPMI complete medium are placed into 24-well tissue culture plates at 2 x 10⁶ cells/well. Cells are incubated 4-6h in the presence or absence of anti-CD3 or appropriate antigen with brefeldin A at 10 µg/ml. Brefeldin prevents exocytosis of proteins and promotes accumulation of the cytokine within the cell. For cytoplasmic cytokine detection, the cells are fixed in 2% paraformaldehyde at room temperature for 5 min following surface staining to distinguish cell subtypes. Cells are washed and re-suspended in 50 µl PBS 0.2% Saponin and 1 µg anti-cytokine antibody and incubated at room temperature for 20 minutes. Next, the cells are washed twice in Saponin and re-suspended in PBS/FCS. The specificity of the cytokine antibody staining is confirmed by pre-blocking the cells with an excess of un-conjugated antibody of the same isotype and cytokine specificity or by incubating the cells in the presence of recombinant cytokine. Phycoerythrin (PE)-labeled irrelevant antibody controls also are included to assess background staining. The cells are analyzed using flow cytometry.

**ELISAs:** ELISAs measure cytokine and antibody concentrations in cell supernatants from cells cultured in microtiter plates and manipulated as describe above. Many of these assays are already operational within our laboratory. Cytokines are assayed using two monoclonal antibodies (mAbs) in a two-site sandwich ELISA. The anti-cytokine mAbs are purified by ammonium precipitation from supernatants of antibody secreting hybridoma clones. Microtiter plates are coated with 50 µl of 1 ug/ml coating antibody in PBS containing Tween 20 (PBS-T), and incubated at 4°C overnight. Then, wells are blocked by the addition of 150 µl of 10% FCS in PBS with incubation at 37°C for 30 min. Standards comprise recombinant cytokine or cytokine-containing supernatants from mitogen or antigen-activated spleen cells from schistosomiasis-infected mice. Sample and standard dilutions are made in RPMI containing 10% FCS (complete RPMI) in a separate 96-well flat bottom microtiter plate, and 50 µl volumes are transferred to the ELISA plates that have been washed three times in PBS-T. Samples are incubated in the assay plates for 1 h at 37°C. Appropriate mAb is conjugated to biotin. After washing three times in PBS-T, each well will receive 50 µl of antibody-biotin conjugate at 0.5 µg/ml in 1% BSA/PBS-T. Plates are incubated at room temperature for 1 h followed by washing three times in PBS-T. Streptavidin-horseradish peroxidase conjugate (75 µl) is added at 1 µg/ml in 1% BSA/PBS-T and incubated at room temperature for 1 h. Plates are washed 10 times in fresh PBS-T, and 100 µl of substrate (2.2'-azino(3-ethylbenzthiazoline sulfonic acid) at 1 mg/ml in 44 mM Na₂HPO₄, 28 mM citric acid, and 0.003% H₂O₂ is added. The colored product is measured at a wavelength of 405 nm with a reference wavelength of 490 nm, using a multiscan microplate reader.

Immunoglobulins are quantified using anti-isotype specific ELISAs. Affinity-purified goat anti-IgM, -IgG1, -IgG2a, -IgG2b, -IgG3, -IgA and -IgE are used as capture antibodies and absorbed to flexible polyvinyl microtiter dishes at 10 µg/ml. After addition of culture supernatants, incubation and washing, appropriate isotype alkaline-phosphatase-conjugated goat anti-Immunoglobulin is used to detect total mouse Immunoglobulin bound to the plates. Standard curves are generated using purified Immunoglobulin. To measure parasite antigen-specific antibody, soluble antigen is biotinylated and used to detect bound mouse Immunoglobulin. The plates are analyzed on a ELISA reader at 410 nm, and concentrations of total Immunoglobulin are determined using the standard curve and best-fit analysis software. Antigen-specific antibody concentrations are compared relative to the O.D. readings, since soluble parasite antigen is not a defined antigen that permits precise quantitation.

**ELISPOT Assays:** ELISPOT assays are established to count lymphocytes secreting either polyclonal antibody or cytokines. Ninety-six-well nitrocellulose-backed microtiter plates are coated overnight at 4°C with 1µg/ml of either anti-Immunoglobulin or anti-cytokine antibodies in PBS-T. The plates then are blocked with PBS containing 10% FCS and washed extensively with PBS-T.

Serial dilutions of a single cell suspension, starting with 5 x 10⁴ cells/well, are incubated on the plate for 5 h at 37°C in a humidified 5% CO₂ atmosphere. The plates are washed with PBS-T and overlaid with biotinylated anti-Immunoglobulin or -cytokine antibodies overnight at 4°C. Next, plates are washed and treated with streptavidin-glucose oxidase-conjugate for 2 h and washed again.

The antibody or cytokine secreted by single cells is visualized with substrate. The colorimetric reaction is halted after 30 min by washing and spots enumerated under 30X magnification. The dilution of cells producing 10-50 spots/well is used to calculate the total number of secreting cells per sample. Controls include wells coated with inappropriate goat antibody or inappropriate antigen, or left uncoated.

A modification of the assay using soluble antigen to coat the wells permits quantitation of parasite antigen-specific, Immunoglobulin secreting B cells also. Briefly, for example, plates are coated with adult *T. Muris* antigen at 0.25 µg/well or appropriate irrelevant control antigen. Cells are added to the wells after washing. After appropriate incubation, the plates are washed again and treated as described above.

### D. Evaluation of Disease Pathology

Evidence of the presence of an autoimmune disease and its cure or amelioration is needed to determine the need for treatment and to monitor treatment progress. The following procedures are utilized to measure the clinical parameters of the noted diseases.

### 1. Inflammatory bowel disease

**Evaluation of Inflammation:** In mice, clinical evidence of disease includes weight loss, diarrhea, rectal prolapse and histological evidence of intestinal inflammation. Thus, improvement in these parameters would signify amelioration of disease.

To grade intestinal inflammation in animal models, tissue is removed, Swiss-rolled and embedded in paraffin according to standard methods. The sections are stained with hematoxylin and eosin. The degree of colonic inflammation is graded semiquantitatively from 0 to 4 in a blinded fashion by a single pathologist using our usual standardized technique: 0=no inflammation; 1=low level inflammation; 2=intermediate level inflammation; 3=high level inflammation with wall thickening; and 4=transmural infiltration, and loss of goblet cells with wall thickening.

To count mast cells, intestinal tissue samples from individual mice are prepared by the Swiss-roll technique, fixed in Camoy's fixative, paraffin embedded and processed for staining with Alcian Blue and safranin. Fifty adjacent fields of a given section are scanned for mucosal mast cells in the lamina propria and muscle layers. Mast cells are identified by their distinctive intracellular granular staining with Alcian Blue. All samples are evaluated blindly.

Disease activity in humans is monitored using various clinical, laboratory and histological criteria. There are several well established IBD disease activity indices that monitor clinical parameters like frequency of diarrhea and abdominal pain. One particularly useful index for the assessment of Crohn's disease is the Crohn's Disease Activity Index, or CDAI (Best et al., 1976, Gastroenterology 70: 439). The CDAI incorporates 8 variables related to the disease activity and has been used in most recent studies of therapeutic agents in Crohn's disease. It includes the number of liquid or very soft stools, the severity of abdominal pain or cramping, general well-being, the presence of extraintestinal manifestations of the disease, presence or absence of an abdominal mass, use of antidiarrheal drugs, hematocrit, and body weight. The composite score ranges from 0 to about 600. Scores below 150 indicate remission and scores above 450 indicate severe illness.

A tested, accepted and disease specific quality of life questionnaire also may be administered prior to and after treatment to assess therapeutic progress. The Irvine Inflammatory Bowel Disease Questionnaire is a 32-item questionnaire (Irvine et al., 1996, The Short Inflammatory Bowel Disease Questionnaire: a quality of life instrument for community physicians managing inflammatory bowel disease. CCRPT Investigators. Canadian Crohn's Relapse Prevention Trial. Am J Gastroenterol. 1996 91(8):1571-8). It evaluates quality of life with respect to bowel function (e.g. loose stools and abdominal pain), systemic symptoms (fatigue and altered sleep pattern), social function (work attendance and the need to cancel social events) and emotional status (angry, depressed, or irritable). The score ranges from 32 to 224, with higher scores indicating a better quality of life. Patients in remission usually score above 170.

Endoscopic, x-ray and histological assessment of intestinal disease activity may also be used for evaluation. C-reactive protein levels and blood cell sedimentation rate may also be monitored as systemic indicators of inflammation.

### 2. Rheumatoid arthritis

### Evaluation of Inflammation:

For mice with collagen-induced arthritis, mice are examined every other day and their paws scored as follows: 0, normal; 1, Erythema and mild swelling confined to the ankle joint or toes; 2. Erythema and mild swelling extending from the ankle to the midfoot; 3, Erythema and severe swelling extending from the ankle to the metatarsal joints; and 4, Ankylosing deformation with joint swelling. These four parameters can be correlated with the histological changes in the arthritic joints. Treatment success results in a decrease in the arthritis score with improvement in the histology. Anthony DD. Haqqi TM.,Collagen-induced arthritis in mice: an animal model to study the pathogenesis of rheumatoid arthritis. Clinical & Experimental Rheumatology. 17(2):240-4, 1999.

For pristane-induced arthritis, joints may be measured with a micrometer to detect swelling. In humans, RA is scored by measuring joint swelling, erythema, limitation of motion and pain. Additionally, synovial fluid may be analyzed for cytokine and inflammatory protein concentrations, and for leukocyte composition and function, according to methods known in the art. Synovial biopsies provide tissue for histological analysis according to methods known in the art. Felson DT. Anderson JJ. Boers M. Bombardier C. Furst D. Goldsmith C. Katz LM. Lightfoot R Jr. Paulus H. Strand V. et al. American College of Rheumatology. Preliminary definition of improvement in rheumatoid arthritis.Arthritis & Rheumatism. 38(6):727-35, 1995 Schiff M. A rationale for the use of summary measurements for the assessment of the effects of rheumatoid arthritis therapies.Clinical Therapeutics. 25(3):993-1001, 2003

### 3. Lupus

### Evaluation of Inflammation:

The normal development and function of the immune system critically depends on the removal of unwanted cells by a process called apoptosis. Cell-to-cell interactions through specific cell surface molecules and their receptors frequently trigger the process. One such system is called FAS and FAS ligand. Mice deficient in either FAS (*LPR*-/-) or FAS ligand (*GLD*-/-) develop an autoimmune disease like lupus. Reilly CM. Gilkeson GS. Use of genetic knockouts to modulate disease expression in a murine model of lupus, MRL/lpr mice. Immunologic Research. 25(2):143-53, 2002.[

Colonies of *LPR* or *GLD* mice are maintained in micro-isolator housing units under specific pathogen-free conditions. These mice can develop autoimmunity spontaneously, but more predictably after artificial induction. To induce disease, 8-wk-old mice are injected with an agent like pristane. Within two months, the mice have autoimmune disease. Many clinical, histological and immunological criteria useful for judging disease induction and amelioration in both mice and humans are well known in the art. Satoh M. Richards HB. Shaheen VM. Yoshida H. Shaw M. Naim JO. Wooley PH. Reeves WH. Widespread susceptibility among inbred mouse strains to the induction of lupus autoantibodies by pristane.Clinical & Experimental Immunology. 121(2):399-405, 2000

### 4. Juvenile Insulin-dependent Diabetes Mellitus (Type 1)

### Evaluation of Inflammation:

The *NOD* mouse develops type 1 diabetes mellitus similar to humans due to autoimmune destruction of the pancreatic β cells. Clinical, biochemical, immunological and histological examination according to methods known in the art allow assessment of disease induction and amelioration in mice. See, e.g., Adorini L. Gregori S. Harrison LC. Understanding autoimmune diabetes: insights from mouse models. Trends in Molecular Medicine. 8(1):31-8, 2002

### 5. Sarcoidosis

### Evaluation of Inflammation:

In the bead embolization model of pulmonary inflammation, antigens (ie. Th1 or Th2) are coupled to Sepharose beads, which are embolized to the lungs of mice via injection into their tail veins. The animals usually are pre-sensitized to the coupled antigen. The immune system of the host mounts a vigorous immune response to the offending bead. These focal inflammatory responses, which can last for several weeks, can be examined histologically for size. Also, they can be isolated from tissue and studied for cell composition and cytokine production. Moreover, hilar lymph nodes and spleens are readily available for experimentation. See, e.g., Kunkel SL. Lukacs NW. Strieter RM. Chensue SW. Animal models of granulomatous inflammation. Seminars in Respiratory Infections. 13(3):221-8, 1998

Sarcoidosis, a disease of humans, usually involves the lung. Determination of sarcoidosis and the extent of the disease may be made according to methods known in the art. Pulmonary function tests can assess lung compliance and function. Also, bronchiolar lavage obtains inflammatory cells that have infiltrated into the bronchial tree during the inflammatory process. These cells can be studied for composition and function. Pulmonary infiltrates and hilar lymphadenopathy are characteristic of sarcoidosis. Thus, periodic chest x-ray or CT scans can help assess disease activity. Serologic tests, such as measurement of angiotensin converting enzyme activity according to methods known in the art, can be used to gauge disease extent and activity. See, e.g., Hunninghake GW. Costabel U. Ando M. Baughman R. Cordier JF. du Bois R. Eklund A. Kitaichi M. Lynch J. Rizzato G. Rose C. Selroos O. Semenzato G. Sharma OP. ATS/ERS/WASOG statement on sarcoidosis. American Thoracic Society/European Respiratory Society/World Association of Sarcoidosis and other Granulomatous Disorders. Sarcoidosis Vasculitis & Diffuse Lung Diseases. 16(2):149-73, 1999*.*

### 6. Multiple Sclerosis

### Evaluation of Inflammation:

Experimental autoimmune encephalomyelitis is induced in susceptible mice by repeated injection of appropriate sensitizing myelin antigens. Mice are assessed clinically according to the following criteria: 0, no disease; 1, tail atony; 2, hind-limb weakness; 3, hind-limb paralysis; 4, hind-limb paralysis and fore-limb paralysis or weakness; 5, moribund. For histological analysis, the spinal cords and brains are removed and fixed in formalin. The paraffin-embedded sections are stained and examined under light microscopy. Dispersed splenocytes and cells from other regions can be studied *in-vitro* as outlined above. These parameters can help measure disease amelioration or improvement, see, e.g., Sewell, Diane, Zing Qing, Emily Reinke, David Elliott, Joel Weinstock, Matyas Sandor, and Zsuzsa Fabry. Immunomodulation of experimental autoimmune encephalomyelitis by helminth ova immunization. International Immunol. 15:59-69, 2003.

In humans, MS disease activity is gauged by monitoring progression and remittence of neurological signs and symptoms. The most widely used outcomes measurement is called The Expanded Disability Status Scale. Cerebral spinal fluid protein composition and cell content analyzed according to methods known in the art also may be used to monitor disease activity. Moreover, serial MRI studies show new gadolinium-enhanced brain lesions. McDonald WI, Compston A, Edan G, Goodkin D, Hartung HP, Lublin FD, McFarland HF, Paty DW, Polman CH, Reingold SC, Sandberg-Wollheim M, Sibley W, Thompson A, van den Noort S, Weinshenker BY, Wolinsky JS .Recommended diagnostic criteria for multiple sclerosis: Guidelines from the International Panel on the Diagnosis of Multiple Sclerosis Ann Neurol 50(1):121-7. 2001. Poser CM, Paty DW, Scheinberg L, et al."New Diagnostic Criteria For Multiple Sclerosis: Guidelines For Research Protocols" Ann Neurol 1983:13:227-231 Statistical Methods: Some data are analyzed by the one sample t-test to determine whether mean values significantly differ from zero. Paired t-test is used to analyze differences between group means. Analysis of variance and Dunnett's t-test is used to analyze multiple comparison data.

### ANIMAL MODELS

The following Table I illustrates non-limiting examples of animal models useful according to the present invention. Additional non-limiting examples may be found in the art, e.g., as described in Current Protocols in Immunology (2001, Eds. John E. Coligan, Ada M. Kruisbeek, David H. Margulies, Ethan M. Shevach, Warren Strober, published by John Wiley & Sons).

**TABLE 1**

| **TREATABLE DISEASES** | **SOME ANIMAL MODELS** |
|---|---|
| 1. Inflammatory Bowel Diseases | TNBS colitis (Neurath et al., 1995, J. of Exp. Med. 182:1281) |
| | IL-2 mutant mice (Ludviksson et al., 1997, J. of Immunol. 158:104) |
| | IL-10 mutant mice (Berg et al., 1996, J. of Clin. Investigation 98:1010) |
| | TCR transgenic mice (Mombaerts et al., 1993, Cell 75:274) |
| | CD45+ T cells transferred into *SCID* mice (Powrie et al., 1994, Immunity 1:553) |
| 2. Rheumatoid Arthritis | Murine pristane-induced arthritis (Stasluk et al., 1997, Immunol. 90:81) |
| | Murine collagen-induced arthritis (Horsfall et al., 1997, J. of Immunol. 159:5687) |
| 3. Insulin-dependent Diabetes (type 1) | *NOD* mouse (Cameron et al., 1997, J. of Immunol 159:4686) |
| 4. Lupus | *(NZWX NZB) F₁ models* (Santiago et al., 1997, J. of Exp. Med. 185:65) |
| | *GRD, LPR* mouse (FAS mutation) (Bhandoola et al., 1994, Int. Rev. of Immunol. 11:231) |
| 5. Sarcoidosis | *Murine Berylliosis* (Pfeifer et al., 1994, Int. Archives of Allergy & Immunol. 104:332) |
| | *M. avium* mouse (Chen et al., 1994, Science 265:1237) |
| 6. Multiple Sclerosis | Experimental allergic encephalomyelitis in mice |
| | Owens T, Sriram S. 1995, Neurol Clin. 13(1):51-73. Review. |
| | Degaonkar et al., 2002, J Magn Reson Imaging. 16(2):153-9. |
| | Duckers et al., 1996, Neuroscience 1(2):507-21. |

### EXAMPLES

The invention is illustrated by the following nonlimiting examples wherein the following materials and methods are employed.

### Example 1. General Methods:

Animals: Colonies of 129/SV IL-10-/- mutant mice, and appropriate control animals are maintained are housed in facilities maintained as a specific pathogen-free environment according to standard methods.

Parasite Maintenance, Animal Infection, Production of Schistosome Eggs: The maintenance of *T. muris* and the method used for infection are as described by Else and Wakelin, 1990, Parasitology, vol. 100, part 3: 479.

Schistosome eggs were harvested from the livers of schistosome-infected hamsters and stored as described by Elliott, 1996, Methods: A Companion to Methods in Enzymology, 9:255. Five infected hamsters yield about 2 x 10⁶ eggs.

Preparation of *T. suis* Eggs: The following process was used in the preparation and harvesting of *T. suis* eggs. Adult T. *suis* worms were isolated from the colon of pigs 7-8 wks after exposure to an experimental inoculation of T *suis* eggs. Embryonated eggs were obtained by culturing adult worms *in vitro,* and then the excreted eggs, separated from the culture medium by centrifugation, were placed into 0.2% potassium dichromate solution at 22°C for 5-6 wks with bubbling to obtain infective first-stage larvae. Eggs were washed twice in sterile water by centrifugation at 1200 x g for 10 min, counted, and re-suspended in the desired amount of saline based on a calculated dose of 2,500. These eggs were stored for use in the subjects. The ova are stable for at least one year in the refrigerator. To assure infectivity, we monitored patients for the appearance of ova in the stool after colonization. The number of ova in the stool is proportional to the intensity of the infestation. Also, from time to time, we infect pigs with our stored ova to assure continued infectivity.

Infection with *M. avium:* Mice were infected by injecting 10⁶ colony forming units (CFU) of *Mycobacterium avium* (ATCC 25291) intraperitoneally. On day 60 of infection, some mice also received 35 *S. mansoni* cercariae to induce dual infection.

Schistosome Infection and Isolation of Ova: Some experiments used mice (18-20g) infected for 8-9 wks with *S. mansoni*. Mice were infected subcutaneously with 40 cercariae from the Puerto Rican strain.

Granuloma Isolation and Dispersal: Granulomas form in schistosome-infected mice because of natural egg deposition, which begins at the 6th wk of infection. *M. avium* also induces granulomas. We study liver granulomas isolated from infected mice as described (Elliott, 1996, supra). Granulomas were dispersed to produce single-cell suspensions. Isolated granulomas were agitated for 35 min at 37°C in a shaker water bath in RPMI medium containing 5 mg/ml collagenase. The residual granulomas were sucked and expelled through a 1 ml syringe to induce further dissociation. The resulting cell suspension was filtered through gauze and washed three times. Cell viability was determined by Eosin-Y exclusion. This protocol resulted in a high yield of viable inflammatory cells that show preserved surface molecule expression.

Spleen and MLN Dispersal: Splenocytes and MLN were dispersed by teasing and washing the tissue through a stainless steel mesh. The contaminating RBC were lysed with H20, and the cells were washed x3 in RPMI before use.

Induction of TNBS Colitis: Colitis was induced by rectal instillation of trinitrobenzesulfonic acid (TNBS) as described by Neurath et al., 1995, J. Exp. Med., 182: 1281. Briefly, control or parasite-exposed BALB/c mice were fasted for 30 hrs then anesthetized with methoxyflurane. A 1.2mm catheter was advanced 4cm into the colon and 0.1 ml of TNBS solution (5mg/ml TNBS (Sigma) in 50% ethanol) was instilled. The animal was held by the tail for 3 minutes to insure uniform contact with colonic mucosa.

Evaluation of Mucosal Inflammation: To grade intestinal inflammation, tissue was removed at the indicated time points, Swiss-rolled and embedded in paraffin according to standard methods. The sections were stained with hematoxylin and eosin. The degree of colonic inflammation was graded semiquantitatively from 0 to 4 in a blinded fashion by a single pathologist using our usual standardize technique (1 9). O=no inflammation, 1 =low level inflammation, 2=intermediate level, 3=high level inflammation with wall thickening, 4=transmural infiltration, loss of goblet cells, wall thickening.

### Cell Isolation and T Cell Enrichment:

Th1, Th2, and regulatory T cells may be isolated according to methods known in the art, e.g., as described in Current Protocols in Immunology (2001, Eds. John E. Coligan, Ada M. Kruisbeek, David H. Margulies, Ethan M. Shevach, Warren Strober, published by John Wiley & Sons)

In one embodiment, single cell suspensions of MLN were prepared by gentle teasing in RPMI 1640 medium (GIBCO, Grand Island, NY). The cells were briefly re-suspended in distilled water to lyse RBC. The MLN then were washed three times in a large volume of RPMI.

Gut LPMC were isolated as described below. Intestinal tissue was washed extensively with RPMI, and all visible Peyer's patches were removed with a scissors. The intestine was opened longitudinally, cut into 5 mm pieces and then incubated in 0.5 mM EDTA in calcium and magnesium free Hanks' for 20 min at 37°C with shaking to release intraepithelial lymphocytes and epithelial cells. This was repeated after thorough washing. Tissue then was incubated 20 min at 37°C in 20 ml RPMI containing 10% FCS, 25 mM HEPES buffer, 2 mM L-glutarmine, 5x10⁵ M β-mercaptoethanol, 1 mM sodium pyruvate, 100 U/ml penicillin, 5 mg/ml gentamycin, and 100 mg/ml streptomycin (all GIBCO) and 1 mg/ml collagenase (Sigma #CO130) At the end of the incubation, the tissue was subjected to further mechanical disruption using a 1 ml syringe. To remove debris, the LPMC preparations were washed through a pre-wet gauze layered in a funnel with RPMI. Then, the LPMC were washed once and were sieved through a pre-wet 2 cm nylon wool column gently packed into a 10 ml syringe. After washing, cells (up to 2 x10⁷) were layered onto a column of Percoll with a 30:70% gradient. Cells were spun at 2200xG at room temperature for 20 min. The LPMC collected from the 30:70 interface were washed and maintained on ice until used. Cell viability was 90% as determined by eosin Y exclusion.

For cell transfer experiments, MLN T cells were isolated by negative selection using the SpinSep enrichment procedure employing antibody-coated, dense particles as described by the manufacturer (#17031, #17032, Stem Cell Technologies, Vancouver, Canada). Flow cytometry was used after each separation to assure appropriate recovery and purity (>98%) of the Thy+ T cells.

### Cell transfer:

MLN cells were isolated from IL10-/- mice colonized 2 wks with *H. polygyrus* or from age-matched healthy IL10-/- mice without such colonization. These unfractionated, dispersed MLN cells (2x10⁷ cells/mouse) or MLN T cells (5x10⁶ cells/mouse) then were transferred into colitic IL10-/- mice 2 days after discontinuation of piroxicam treatment by ip injection. The colitis was evaluated 14 days later.

### Additional methods for regulatory T cell preparation

In some embodiments of the present disclosure, regulatory T cells may be prepared from cultured cells or an animal for further analysis accroding to methods known in the art, e.g., as described in US Patent Applications with Serial Nos. 2003/0170648, 2003/0157057, 2003/0133936, 2003/0064067, 2003/0049696, 2002/0182730, 2002/0090724, 200210090357.

In vivo sources of cell populations useful as a source of cells include, but are not limited to peripheral blood, leukopheresis blood product, apheresis blood product, peripheral lymph nodes, gut associated lymphoid tissue, spleen, thymus, cord blood, mesenteric lymph nodes, liver, sites of immunologic lesions, e.g. synovial fluid, pancreas, cerebrospinal fluid, tumor samples, and the like. The donor is preferably human, and can be fetal, neonatal, child, adult, and may be normal, diseased, or susceptible to a disease of interest.

For example, the regulatory T cells can be enriched on the basis of expression of cell surface markers. In one embodiment, the cells are positively selected for expression of CD4 and CD25, and can be negatively selected for the absence of CD45RA. Optionally, other markers can be used to further separate subpopulations of the Treg cells, including CD69, CCR6, CD30, CTLA-4, CD62L, CD45RB, and CD45RO. The methods can include further enrichment or purification procedures or steps for cell isolation by positive selection for other cell specific markers.

In another embodiment, regulatory T cells are separated from a complex mixture of cells by techniques that enrich for cells having the characteristics of being CD4⁺CD25⁺, and optionally CD45RA⁻. For isolation of cells from tissue, an appropriate solution may be used for dispersion or suspension. Such a solution will generally be a balanced salt solution, e.g. normal saline, PBS, Hanks balanced salt solution, etc., conveniently supplemented with fetal calf serum, BSA, normal goat serum, or other naturally occurring factors, in conjunction with an acceptable buffer at low concentration, generally from 5-25 mM. Convenient buffers include HEPES, phosphate buffers, lactate buffers, etc.

In another embodiment, separation of regulatory T cell population then uses affinity separation to provide a substantially pure population. Techniques for affinity separation may include magnetic separation, using antibody-coated magnetic beads, affinity chromatography, cytotoxic agents joined to a monoclonal antibody or used in conjunction with a monoclonal antibody, e.g. complement and cytotoxins, and "panning" with antibody attached to a solid matrix, e.g. plate, or other convenient technique. Techniques providing accurate separation include fluorescence activated cell sorters, which can have varying degrees of sophistication, such as multiple color channels, low angle and obtuse light scattering detecting channels, impedance channels, etc. The cells may be selected against dead cells by employing dyes associated with dead cells (propidium iodide, LDS). Any technique may be employed which is not unduly detrimental to the viability of the selected cells.

The affinity reagents may be specific receptors or ligands for the cell surface molecules indicated above. In addition to antibody reagents, peptide-MHC antigen and T cell receptor pairs may be used; peptide ligands and receptor; effector and receptor molecules, and the like. Antibodies and T cell receptors may be monoclonal or polyclonal, and may be produced by transgenic animals, immunized animals, immortalized human or animal B-cells, cells transfected with DNA vectors encoding the antibody or T cell receptor, etc. The details of the preparation of antibodies and their suitability for use as specific binding members are well-known to those skilled in the art.

Of particular interest is the use of antibodies as affinity reagents. Conveniently, these antibodies are conjugated with a label for use in separation. Labels include magnetic beads, which allow for direct separation, biotin, which can be removed with avidin or streptavidin bound to a support, fluorochromes, which can be used with a fluorescence activated cell sorter, or the like, to allow for ease of separation of the particular cell type. Fluorochromes that find use include phycobiliproteins, e.g. phycoerythrin and allophycocyanins, fluorescein and Texas red. Frequently each antibody is labeled with a different fluorochrome, to permit independent sorting for each marker.

In one embodiment, the antibodies are added to a suspension of cells, and incubated for a period of time sufficient to bind the available cell surface antigens. The incubation will usually be at least about 5 minutes and usually less than about 30 minutes. It is desirable to have a sufficient concentration of antibodies in the reaction mixture, such that the efficiency of the separation is not limited by lack of antibody, i.e. using a saturating amount of antibody. The appropriate concentration can also be determined by titration. The medium in which the cells are separated will be any medium which maintains the viability of the cells. A preferred medium is phosphate buffered saline containing from 0.1 to 0.5% BSA. Various media are commercially available and may be used according to the nature of the cells, including Dulbecco's Modified Eagle Medium (dMEM), Hank's Basic Salt Solution (HBSS), Dulbecco's phosphate buffered saline (dPBS), RPMI, Iscove's medium, PBS with 5 mM EDTA, etc., frequently supplemented with fetal calf serum, BSA, HSA, etc.

The staining intensity of cells can be monitored by flow cytometry, where lasers detect the quantitative levels of fluorochrome (which is proportional to the amount of cell surface antigen bound by the antibodies). Flow cytometry, or FACS, can also be used to separate cell populations based on the intensity of antibody staining, as well as other parameters such as cell size and light scatter. Although the absolute level of staining may differ with a particular fluorochrome and antibody preparation, the data can be normalized to a control.

The labeled cells are then separated as to the expression of CD4 and CD25. The separated cells may be collected in any appropriate medium that maintains the viability of the cells, usually having a cushion of serum at the bottom of the collection tube. Various media are commercially available and may be used according to the nature of the cells, including dMEM, HBSS, dPBS, RPMI, Iscoves medium, etc., frequently supplemented with fetal calf serum.

Compositions highly enriched for human Treg activity may be achieved in this manner. The subject population will be at or about 70% or more of the cell composition, and usually at or about 90% or more of the cell composition, and may be as much as about 95% or more of the cell population. The enriched cell population may be used immediately. Cells can also be frozen, although it is preferable to freeze cells prior to the separation procedure, or may be frozen at liquid nitrogen temperatures and stored for long periods of time, being thawed and capable of being reused. The cells will usually be stored in DMSO and/or FCS, in combination with medium, glucose, etc. Once thawed, the cells may be expanded by use of growth factors, antigen, stimulation, dendritic cells, etc. for proliferation and differentiation.

### Cell culture:

For cytokine analysis, cells were cultured for 48h in 96 well microliter plates (Coming, Cambridge, MA) with 200 µl of medium (5 x 10⁵ ells/well) at 37°C. The culture medium was RPMI containing 10% FCS, 25 mM HEPES buffer, 2 mM L-glutamine, 5 x 10⁵ Mβ-mercaptoethanol, 1 mM sodium pyruvate, 100 U/ml penicillin, 5 mg/ml gentamycin, and 100 mg/ml streptomycin (all GIBCO). For most experiments, the cells were cultured alone or with anti CD3 (2Cl1, ATCC) and anti-CD28 mAb (Pharminogen, San Diego, CA)(each at 1 µg/ml). Isolated T cells were cultured in wells previously coated overnight with anti-CD3 and -CD28 mAb. For IL12 analysis, cells were cultured with the synthetic phosphorothioate backbone oligonucleotide ODN 1826 (TCCATGACGTTCCTGACGTT) (SEQ ID NO. 3) that contains two (underlined) immunostimulatory CpG motifs (13;14), provided by the Coley Pharmaceutical Group (Wellesley, MA), and used at 0.6 µg/ml to stimulate production.

ELISAs for Murine Cytokines: ELISAs were performed as described in section VI, above.

### Example 2. Th2 response to S. mansoni down-modulates an ongoing Th1 response to an unrelated bacterial Th1-inducing antigen:

It is well established that Th cell immune responses can polarize into Th1 or Th2 patterns. This polarization occurs because IFNγ from Th1 cells inhibits proliferation of Th2 cells, while IL-4 and IL-10 from Th2 cells inhibits development of Th I cells.The following experiments demonstrated that schistosomiasis alters the murine Th1 response to an established mycobacterial infection.

Mice were co-infected with *M avium and S. mansoni* to evaluate the host response to these distinctly different Th1 and Th2 inflammatory stimuli. BALB/cAnN mice develop chronic *M. avium* infection when injected with this organism (10⁶ CFU). Sixty days after establishment of the mycobacterial infection, the mice were infected with *S. mansoni* (40 cercariae). The mice were killed sixty days later. Control groups included mice receiving either *M. avium* only for 120 days or *S. mansoni* only for 60 days. Dispersed splenocytes or isolated granuloma cells from these animals were cultured *in vitro* (4 x 10⁵ cells/well) for 48h in the presence or absence of schistosome egg antigen (SEA, a strong Th2 antigen) or mycobacterial antigens purified protein derivative (PPD, a strong Th1-inducing antigen) used at optimal concentration. After the incubation, supernatants were assayed for cytokine or immunoglobulin production using ELISAS. The data in figures 1-3 are mean values +/- SD of three separate experiments.

Splenocytes from mice infected only with *M. avium* secreted large amounts of IFNγ following stimulation with PPD (Thl antigen). Spleen cells from uninfected control mice produced none. Most importantly, no IFNγ was detected in spleen cell cultures from concurrently infected mice (*M. avium* alone vs. concurrent infection, P<.001, Figure 1). Soluble schistosome egg antigen (SEA, Th2 antigen) stimulated only IL-4 and IL-5 release from splenocytes of *S. mansoni-infected* animals. Mice singularly infected with *M. avium* produced no IL-4 or IL-5 in response to PPD or SEA. However, splenocytes from co-infected animals secreted some IL-4 following PPD stimulation (Figure 1).

Granulomas were isolated from the livers of mice infected with *M. avium or S. mansoni,* or from animals that had concurrent infection. Concurrently infected animals develop liver granulomas that contain both schistosome eggs and mycobacteria readily evident on histological examination. Dispersed granuloma cells from these animals were cultured *in vitro* for 48h in the presence or absence of SEA or PPD used at optimal concentration. Granuloma cells from mice only infected with *M.avium* secreted large amounts of IFNγ following stimulation with PPD. No IFNγ was detected in granuloma cell cultures from concurrently infected mice *(M. avium* alone *vs* other, P<.001) (Figure 2). SEA stimulated IL-4 release from granuloma cells of *S. mansoni-*infected animals. SEA did not promote IFNγ secretion under any circumstance. Mice singularly infected with *M.avium* produced no IL-4 in response to PPD. However, granuloma cells from co-infected animals secreted some IL-4 following PPD stimulation (Figure 2).

Th1 responses promote IgG2a production, whereas Th2 reactions enhance IgG1 and IgE. Figure 3 shows that mice infected with *M*. *avium* have high serum IgG2a levels. Yet, co-infected animals have normal serum IgG2a concentrations, but increased IgG1 and IgE levels. These data taken together show that a Th2 response to a helminthic infection can down-modulate the ongoing host response to even a strong Th1-inducing organism like *M. avium.*

### Example 3. Colonization with intestinal helminths or exposure to their ova attenuates Th1-type gut inflammation in murine TNBS-induced colitis:

Rectal instillation of TNBS in 50% ethanol induces a colitis in mice that shares features with Crohn's disease. The colonic inflammation is characterized by infiltrating CD4+ T cells with elevated IFNγ mRNA expression. Lamina propria T cells from TNBS treated mice secrete 50 fold more IFNγ and 5 fold less IL4 than T cells from controls (Neurath et al., 1995, supra). Lamina propria mononuclear cells secrete 30 fold more TNFα than cells from control mice (Neurath et al., 1997, Eur. J. Immunol., 27: 1743). Importantly, TNBS colitis can be prevented or improved by treatment with anti-IL-12 (Neurath et al., 1995, supra), anti-TNFα (Neurath et al., 1997, supra), or rIL-10 (Duchmann et al., 1996, Eur. J. Immunol., 26: 934). TNBS-induced colitis also can be prevented by previous oral exposure to the hapten (Elson et al., 1996, J. Immunol., 157: 2174) probably by increasing mucosal IL-4, IL-10 and TGFβ responses (Neurath et al., 1996, J. Exp. Med., 183: 2605).

TNBS colitis model using BALB/c mice is established. Rectal administration of TNBS (0.1 ml of 5 mg/ml stock) in 50% ethanol reproducibly produced colitis in these animals. For each of the TNBS experiments discussed below, parasite-exposed and control animals were administered the same preparation of TNBS on the same day by the same operator who was blinded to the treatment group.

### A. Schistosomiasis inhibits IFNγ release from MLN and spleen cells of TNBS treated mice.

It was investigated whether schistosome infection alters the Th1 response in TNBS treated animal colitis model mice. Mice were infected with 35 *S. mansoni* cercariae by sc. injection. Worms mature and begin to lay eggs about 6 weeks after initiation of infection. Two weeks later (at 8 wks of infection), mice were treated with TNBS. The capacity of MLN and spleen cells to make IFNγ in response to T cell stimulation (anti-CD3) was examined several days later. As shown in Table 2, natural schistosome infection strongly inhibits IFNγ release from mesenteric lymph node (MLN) and spleen cells of TNBS-treated mice.

| Table 2. | | |
|---|---|---|
| Schistosomiasis Inhibits IFNγ Production from MLN and Splenic T Cells of TNBS-Treated Mice | | |
| Group | α-CD3 stimulated IFNγ (ng/ml) | |
| | MLN cells | Spleen cells |
| Uninfected mice given TNBS | 3.2 ± 0.7 | 44.1 ± 2.3 |
| Schistosome-infected mice given TNBS | 1.9 ± 0.3^{a} | 4.2 ± 0.4^{b} |
| Mean IFNγ (ng/ml) ± SE of triplicate determinations measured by ELISA. a) p<0.1 b) p<0.05 Cultures contained 10⁶ dispersed MLN or Spleen cells/well incubated in 200 µl medium for 48 hrs at 37°C in the presence of anti-CD3 (1 µg/ml) (2C11). Results are representative of two experiments. | | |

### B. Exposure to schistosome ova inhibits IFNγ release from MLN and spleen cells of TNBS treated mice.

In schistosomiasis, it is exposure to the parasite ova rather than adult worms that induces a Th2 response. Schistosome infection does not induce strong Th2 responses until the worms mature and begin to lay eggs (Grzych et al., 1991, J. Immunol., 146: 1322). Mice exposed to intact schistosome eggs in the absence of natural infection develop a strong Th2 response (Oswald et al., 1994, J. Immunol., 153: 1707). These observations suggest that exposure to schistosome eggs in the absence of natural infection may induce Th2 and suppress Th1 responses.

To examine whether pre-exposure to schistosome eggs would inhibit Th1 responses without requiring infection by adult worms, mice were inoculated twice with 10⁴ schistosome eggs by intraperitoneal (ip) injection at 14 and 4 days prior to rectal challenge with TNBS. These times were chosen to model the continuous egg deposition that occurs in natural infection. Mice not exposed to parasite eggs but treated with TNBS served as controls. The eggs were previously frozen and were not viable at the time of injection. Once again, the capacity of MLN and spleen cells to make IFNγ in response to T cell stimulation (anti-CD3) was examined several days after TNBS instillation. Like natural schistosome infection (Table 1), ip. egg exposure inhibited IFNγ production from MLN and splenic T cells of TNBS-treated mice as shown in Table 3.

| **Table 3.** | | |
|---|---|---|
| Exposure to Schistosome Eggs Inhibits IFNγ Production from MLN and Splenic T Cells of TNBS-Treated Mice | | |
| Treatment | α-CD3 stimulated IFNγ (ng/ml) | |
| | MLN cells | Spleen cells |
| TNBS alone | 4.65 ± 0.02 | 47.1 ± 3.0 |
| Eggs ip. and TNBS | 2.19 ± 0.11^{a} | 21.8 ± 3.4^{b} |
| Mean IFNγ (ng/ml) ± SD of triplicate determinations measured by ELISA. a) p<0.5. Cultures contained 10⁶ dispersed MLN or spleen cells/well incubated in 200 µl medium for 48 hrs at 37°C in the presence of anti-CD3 (1 µg/ml) (2C11). Results are representative of three separate experiments. | | |

### C. Exposure to schistosome ova protects mice from TNBS-induced colitis.

Inhibiting development of the mucosal Th1 response can attenuate TNBS-induced colitis. Prior exposure to schistosome eggs inhibits Th1 cytokine secretion by MLN and splenic T cells. To examine if ip injection of schistosome eggs would inhibit TNBS-induced colitis, eggs were injected as above followed by TNBS treatment. Egg treatment dramatically reduced cumulative mortality in three separate experiments from 60% (16/27) in the control group to 22% (6/27) in egg-exposed mice. Intestinal inflammation was scored on a 4-point scale as detailed in General Methods, above. In the mice that survived, egg treatment attenuated intestinal inflammation from 3.1 ± 0.5 (mean ± SD) in the control group to 1.3 ± 0.3 in the egg exposed mice (p<0.05, Figure 4). Subsequent experiments showed that the largest difference between groups was evident at 3 days after TNBS treatment. Other experiments carried out to 14 days after TNBS instillation showed that egg exposure afforded prolonged protection. These data indicate that schistosome eggs protect mice from developing fatal colitis by inhibiting mucosal Th1 responses.

### D. Intestinal helminths induce host Th2 responses.

Whether helminthic parasites other than *S. mansoni* can modulate host Th1 responses is examined. Expression of protective immunity to intestinal nematodes is CD4 T cell-dependent. Mice expel worms or limit infection by mounting a Th2 response. Worm expulsion does not appear to exclusively depend on intestinal eosinophilia nor mucosal mastocytosis. IL-4 may have a critical role in worm expulsion, since treatment with blocking anti-IL-4 or anti-IL-4 receptor mAb promotes worm retention (Else et al., 1994, J. Exp. Med., 179: 347). Conversely, treatment with IL-4 promotes worm clearance (Figure 4).

*T. muris* lives in the colon of the murine host. It is related to *Trichuris trichiura,* a parasite carried by nearly one billion people during their lives (Grencis et al., 1996, Gastroenterology Clinics of North America, 25: 579). Ingestion of eggs initiates infection. The eggs release larvae that penetrate the cecal epithelium. They then mature into adult worms. The parasite does not replicate within the host allowing us to control the intensity of infection (Bancroft et al., 1994, Eur. J. Immunol., 24: 3113).

*T. muris* was used to down-modulate intestinal Th1 responsiveness. *T. muris* infection is established by oral gavage with 250 embryonated eggs containing viable larvae. BALB/c mice can harbor *T. muris* and naturally expell the worms within 4 wks of infection. *T. muris- or* sham-infected mice were treated with rectal instillation of TNBS 4 wks after initiation of infection. Prior colonization with *T. muris* reduced cumulative mortality in two separate experiments from 58% (7/12) in the sham infected group to 21% (3/14) in the parasite exposed group. Furthermore, mice previously colonized with *T. muris* develop attenuated TNBS colitis (0.92 ± 0.5, mean ± SD) as compared to sham-infected mice (3.13 ± 0.63, p<0.05). These data indicate that prior exposure to intestinal parasites *(T. muris)* protect mice from developing severe Th1 -mediated colitis.

### Example 4. IL-10 gene disruption does not significantly alter the host/parasite interaction.

IL-10 is an important immunoregulatory cytokine that down modulates macrophage activation and accessory cell function (Moore et al., 1993, Ann. Rev. Immunol., 11: 165). Mice rendered IL-10 deficient by targeted gene disruption (IL-10-/-) develop a chronic enterocolitis that is influenced by colonic flora (Kuhn et al., 1993, Cell, 75: 263). The intestinal inflammation is attenuated by treatment with anti-IFNγ antibody demonstrating that the colitis results from overly exuberant Th1 responses to colonic contents (Berg, et al., 1996, J. Clin. Invest., 98: 1010). These mice serve as excellent models for spontaneous colitis similar to that of Crohn's disease. In this example IL-10 -/- mice on the 129 and C57B1/6 backgrounds were used.

### A. IL-10-/-mice can mount Th2 responses to parasites.

Mice infected with the intestinal nematode *N*. *brasiliensis* develop Th2-type inflammation to the parasite with production of IL-4, IL-5 and IL-10. *N. brasiliensis* can colonize IL-10-/- mice and stimulate an appropriate intestinal Th2 response (Kuhn et al., 1993, supra). IL-10 -/- mice were infected with *S. mansoni* to examine whether they mount a TH2 response.

Table 4 shows that splenocytes from IL-10 gene-disrupted mice colonized for 8 wk with *S. mansoni* secrete large amounts of IL-4 upon stimulation with schistosome egg antigen (SEA) or anti-CD3. The granulomas that surround schistosome eggs in these mice contain the usual high percentage (45-50%) of eosinophils, and make IL-4 and IL-5. Thus, they show an effectiveTh2 response. These data indicate that exposure to helminthic parasites like *S. mansoni* will induce a strong Th2 response even in the absence of IL-10.

| Table 4. | | | | |
|---|---|---|---|---|
| Schistosomiasis Induces a Th2 Response in IL10 Deficient Mice | | | | |
| | | Spleen Cells from a Schistosome-infected Mice | | |
| Cytokine | | Unstimulated | SEA-Stimulated | α-CD3 Stimulated |
| IL-4 (ng/ml) | | | | |
| | Wild Type | 0.07 ± 0.08 | 0.21 ± 0.02 | 3.94 ± 0.12 |
| | IL-10-/- | 0.41 ± 0.10 | 2.56 ± 0.23 | 5.06 ± 0.33 |
| Mean IL-4 (ng/ml) ± SD of triplicate determinations as measured by ELISA. Cultures contained 10⁶ cells/well incubated for 48 hrs at 37°C in 200 µl medium in the absence or presence of schistosome egg antigen (SEA 5 µg/ml) or anti-CD3 (1 µg/ml) (2C11). Results are representative of two separate experiments using a minimum of four wild type and KO mice in each experiment. | | | | |

### B. Helminthic Th2-conditioning inhibits natural development of mucosal inflammation in IL-10 -/- mice.

IL-10 deficient mice could harbor helminthic parasites and mount a strong Th2 response. Because IL-10 -/- mice can develop Th2 responses and harbor intestinal parasites, they serve as excellent models to study the effect of parasite exposure on spontaneous or ongoing colitis. IL-10 is an important anti-inflammatory cytokine. It is possible that disruption of this essential immunoregulatory circuit will prevent mucosal Th2 conditioning by parasites. Present evidence indicates that infection with *T. muris* impedes the spontaneous colitis that develops in IL- 10-/- mice. Animals (6-wk-old) received *T. muris* or sham infection and were killed 6 wks later. Colonic inflammation of *T. muris* or sham-infected IL-10-/- mice was scored on a 4-point scale as detailed in General Methods, above. Prior infection with *T. muris* attenuated intestinal inflammation from 3.0 ± 0.3 (mean ±SE) in the sham-infected group to 2.2 ± 0.1 (p<0.05) in the parasite exposed IL-10-/- mice. These data demonstrate that prior exposure to helminthic parasites attenuates spontaneous colitis in IL-10 deficient mice.

### Example 6. Intestinal colonization with Trichuris suis down-modulates disease activity in patients with Crohn's disease:

Patients with Crohn's disease are conolozed with *Trichuris suis* and assessing improvement in disease activity.*T.suis,* the porcine whipworm, is closely related to *T*. *trichiura,* a human intestinal helminth common in underdeveloped countries. The whipworm is a potential agent for therapy. The natural human parasite *Trichuris trichiura* is a very small organism that resides in the colon by attachment to the mucosa. Ordinary colonization usually produces no symptoms and causes no health problems for the host. This is the case in millions of colonized people throughout the world, but in a minority, heavy infestation produces diarrhea, bleeding and iron deficiency anemia. It is of interest that the parasite's life cycle is such that the host is not self-infected. The eggs would require a soil phase for maturation to become infective and then must be re-ingested to increase the parasitic load for an individual. Thus, *T. trichiura* infestation will not increase within the host unless eggs in the soil are ingested. The agent is readily and effectively treated with three days of mebendazole. The human whipworm could be used to colonize the host and be considered as an experimental agent to modify the immune process in Crohn's disease.

However, *Trichuris trichiura* poses a potential health problem for the host. Because the human is the only natural host, eggs for experimental use would have to be harvested from other humans, creating the potential for transmission of other human infectious diseases to the experimental subject. Therefore, a closely related animal parasite was used. The porcine whipworm *(Trichuris suis)* has the potential to temporarily colonize a human host without causing symptoms, disease, a co-infectious disease or a public health hazard.

The porcine whipworm is closely related to the species infecting humans. The two organisms are of the same family and are morphologically similar, but they belong to a different species and can be distinguished morphologically, developmentally and clinically. The porcine whipworm ovum is slightly larger, has a different shaped spine, and the rate of development from an egg to an adult is slower than that of *Trichuris trichiura* (Beer, 1976, Res. Vet. Sci., 20:47). Importantly, we can obtain infective parasite ova from SPF animals.

A supply of infective *T. suis* eggs was produed as described above. The eggs were tested to confirm absence of contamination with enteric pathogens *(e.g. Shigella, Salmonella, Campylobacter, Yersinia,* and enterotoxic *E. coli*) and viruses (e.g. CMV, HSV, VZV, adenovirus and enteroviruses).

Two patients with advanced, medication-resistant Crohn's disease were colonized with *T. suis.* They tolerated the parasite with little or no symptoms attributable to this organism. Table 5 shows that after achieving patent colonization, both participants had a drop in CDAI values, diarrhea, and inflammatory indices. This result indicates that helminths are useful for modulating abnormal immune responses, including, but not limited to Crohn's disease.

| Table 5. | | | | |
|---|---|---|---|---|
| Patients with Crohn's disease may benefit from colonization with *T. Suis.* | | | | |
| Patient | Parameter | Baseline ^{a} | Week 2 ^{b} | Week 4 ^{b} |
| 1 | CDAI^{c} | 250.2 | 269.9 | 147.4 |
| | Stools/week | 67 | 101 | 49 |
| | ESR^{d} | 17 | 11 | 4 |
| | C-reactive protein | <0.5 | <0.5 | <0.5 |
| 2 | CDAI | 341.3 | 169.6 | 136.7 |
| | Stools/week | 77 | 27 | 25 |
| | ESR | 19 | 7 | ND |
| | C-reactive protein | 0.9 | <0.5 | <0.5 |
| a) Stable values at entry into study. b) Values at 2 and 4 weeks after initiation of colonization with *T. suis.* c) Crohn's disease activity index. d) Erythrocyte sedimentation rate. ND; not determined. | | | | |

### Example 7. H. polygyrus in the prevention and therapy of inflammatory bowel disease:

To investigate the role of regulatory t cells in parasite induced protection against inflammatory disease, more experiments were conducted.

The experiments used the murine intestinal helminth *H. polygyrus. H. polygyrus* inhabits the duodenum of the host without causing inflammation or injury to the terminal ileum or colon. TNBS in alcohol induces colitis when instilled intra-rectally into mice. Fig 5 shows that this organism protects mice from TNBS-induced colitis. Colonization was established by oral gavage of 200 viable larvae. The mice expel the worms about 2 wks after initiation of the colonization. *H. polygyrus-* or sham-infected mice were treated with rectal instillation of 0.1 ml of TNBS (5mg/ml in 50% EtOH) 4 wks after initiation of worm colonization. Inflammation was assessed in stained histological sections 3 days after induction of the colitis.

Experiments were condicted to test if helminthic colonization protects mice from TNBS-induced IBD by limiting intestinal production of Th1 cytokines like IFNγ, TNFα and IL12. Fig 6 and 7 show that exposing healthy WT mice free of IBD to *H. polygyrus* renders the distal intestinal mucosa less capable of IFNγ and IL12 production,

Regulatory cytokines like IL10, TGFβ and PgE2 are capable of restricting "Th1" cell function. It was determined if *H. polygyrus* promotes production of these regulatory cytokines within the intestinal mucosa. Fig 8 shows that this worm induces mucosal IL 10 production. IL10 down-regulates an array of proinflammatory mediators like TNFα, IL6 and IL12. Therefore, it is reasonable to assume that IL10 produced in intestinal mucosa restricts "Th1" cell function. Fig 9 shows that LPMC from mice with *H. polygyrus* make substantially more IFNγ when cultured with inhibitory anti-IL10R mAb supporting this postulate.

Potentially important immunoregulatory cytokines that can protect from disease include prostaglandin E2 (PgE2), IL4, IL5, IL13 and TGFβ. Figures 10-12 show that helminths induce produce of protective cytokines E2 (PgE2), IL4, IL5, IL13 and TGFβ in the intestinal mucosa.

T cells are the major source of IFNγ in the intestinal mucosal (Fig 13). IFNγ is important for induction of TNBS colitis.

Intestinal helminths induce regulatory T cells in the MLN that migrate to the intestine to locally control mucosal immune reactivity. The experiments revealed (Fig 14) that LPMC from naïve uninfected WT mice display diminished capacity to produce IFNγ after transfer of MLN T cells from worm-infected mice.

Some experiments used green fluorescent protein (GFP) transgenic mice for adoptive transfer experiments to determine if the MLN regulatory T cell subsets entered the intestinal lamina propria to subsequently produce regulatory cytokines. As seen in Fig 15, putative MLN regulatory T cells from GFP+ mice bearing *H. polygyrus* do enter the intestinal mucosal of healthy WT recipients.

This experiment shows how helminths protect from disease. It was found in clinical trials that helminthic colonization improves active IBD. How colonization with *H. polygyrus* reverses ongoing murine colitis was also tested.

IL10-/- mice develop chronic Th1 colitis that worsens gradually as animals grow older. However, as true for most IBD models of spontaneous disease, IL10-/- mice in a SPF facility develop colitis with variable expression only after several months. The IBD model was improved to make it more useful for experimentation. It was found that feeding 5-wk-old IL10-/- mice an NSAID (piroxicam) for 2 wks induces severe Th1 colitis that persists indefinitely even after the drug is stopped. The inflammation is highly predictable from mouse-to-mouse, involving the colon and TI with uniform severity (Fig 16). WT mice do not develop colitis with piroxicam treatment.

It was found that colonization of IL10-/- mice with *H. polygyrus* resolves previously established colitis (Fig 17). Five-wk-old C57BL/6 IL10-/- mice received piroxicam (80mg/250g feed). After 2 wks, piroxicam was stopped. At this time point, all mice had colitis. Two days after piroxicam was stopped, some mice were colonized with 200 *H. polygyrus* by gastric gavage, whereas others were sham-treated. After 2 wks of colonization, the colitis score fell from 3.6±0.4 (SE) in controls to 0.55±0.5 in colonized mice (p=0.001).

Healing of established colitis by *H. polygyrus* is associated with changes in LPMC cytokine production.

LPMC isolated from naïve (no piroxicam) or colitic sham-colonized mice release copious amounts of IFNγ upon stimulation with anti-CD3/anti-CD28 (Fig 18) and IL12 upon stimulation with CpG oligonucleotide (0.6µg #1826/ml). LPMC from piroxicam-treated *H. polygyrus-*colonized IL10-/- mice make little to no IFNγ detectable by sensitive (30pg/ml) ELISA and have curtailed release of IL12 (Fig 18). LPMC from naïve or colitic IL10-/- mice make no IL4 and little IL13 (Fig19). In contrast, LPMC from *H. polygyrus*-colonized mice make some IL4 and copious amounts of IL13. Helminthic colonization reverses colonic inflammation, but the LPMC cytokine profile does not simply return to that of naive IL10-/- mice. Rather, *colonization results in a novel cytokine profile associated with healing of colitis.*

*H. polygyrus* colonization activates regulatory circuits in LPMC of IL10-/- mice. Mixing IFNγ-producing LPMC from colitic IL10-/- mice 1:1 with LPMC from *H. polygyrus*-colonized mice, results in co-cultures with inhibited IFNγ production.

*H. polygyrus* resides in the duodenum, but initiates regulatory circuits that reverse colitis and alter distant LPMC cytokine profiles. It was showed that helminth colonization induces or activates circulating regulatory cells that reside within the MLN T cell compartment of *H. polygyrus*-colonized mice. Transfer of 20x10⁶ MLN cells or 5x10⁶ MLN T cells from *H*. *polygyrus*-colonized IL 10-/- mice into animals with established IL10-/- colitis results in resolution of the inflammation (Fig 20).

Scurfin is a transcription factor, encoded by the *foxp3* gene, important for development or activity of regulatory T cells. *Foxp3* transcripts are increased in MLN of *H. polygyrus*-colonized compared to worm-free IL10-/- mice as quantified by real-time RT-PCR (Fig 21). Each decrease in PCR cycle threshold is equivalent to a doubled *Foxp3* content normalized to HPRT mRNA. Helminthic colonization results in 3- to 5-fold increased *Foxp3* expression in MLN cells. *Foxp3* also is increased (3-fold) in LPMC of *H. polygyrus*-colonized IL10-/-mice compared to worm-free controls. Only regulatory T cells are known to express *Foxp3* (Hori, S., T. Nomura, and S. Sakaguchi. 2003. Control of regulatory T cell development by the transcription factor Foxp3. Science. 299:1057-1061). *The increase in Foxp3 expression in MLN and LPMC supports the hypothesis that helminths induce regulatory T cells that orchestrate resolution of established colitis.*

Other transcripts for immunoregulatory factors are altered by colonization with *H. polygyrus. Smad7* is a transcription factor that blocks TGFβ signaling. Colonization with *H. polygyrus* decreases MLN cell *Smad7* expression 4.5-fold as measured by real-time RT-PCR (Fig 22). *Smad7* transcripts also are decreased in LPMC. A decrease in *Smad7* would permit cells to be more responsive to immunoregulatory TGFβ. Furthermore, the decrease in *Smad7* expression shows that multiple regulatory mechanisms likely are functioning to resolve IL10-/- colitis.

The primers and probe for Smad7 are TCCTGCTGTGCAAAGTGTTC (SEQ ID NO. 4), GAGTAAGGAGGAGGGGGAGA (SEQ ID NO. 5), and FAM-TTGATCTTCCCGTAAGATTCACAGCAACA-TAMRA (SEQ ID NO. 6). The expression of Foxp3 and Smad7 mRNA are normalized to that of HPRT. The primers and probe for HPRT are TGAAGAGCTACTGTAATGATCAGTCAAC (SEQ ID NO. 7), GCAAGCTTGCAACCTTAACCAT (SEQ ID NO. 8), and TET-TGCTTTCCCTGGTTAAGCAGTACAGCCC-TAMRA (SEQ ID NO. 9).

### OTHER EMBODIMENTS

The foregoing examples demonstrate experiments performed and contemplated by the present inventors in making and carrying out the invention. It is believed that these examples include a disclosure of techniques which serve to both apprise the art of the practice of the invention and to demonstrate its usefulness.

## Claims

1. An *in vitro* method of screening for a helminthic parasite preparation that alters the level of a human regulatory T cell activity, said method comprising the steps of:
(a) obtaining a helminthic parasite preparation;
(b) contacting said helminthic parasite preparation with a human *in vitro* system containing regulatory T cells; and
(c) determining the level of an internal marker for regulatory T-cell activity in said target after said contacting, wherein said internal marker is the transcription factor FOXP3 (Scurfin); and
wherein a change in said level of said internal marker after said contacting is indicative of said helminthic parasite preparation altering a regulatory T cell activity.

2. The method of claim 1 wherein said level of said transcription factor is measured at its protein or mRNA level.

3. The method of claim 1, wherein said regulatory T cell mediated activity is found in the lamina propria mononuclear cells of said system.

4. The method of claim 1, wherein said regulatory T cell mediated activity is found in the mesenteric lymph nodes of said system.

5. A method for monitoring the treatment efficacy of a helminthic parasite preparation for an autoimmune or allergy disease in a non-human animal model comprising:
(i) administering a composition comprising a helminthic parasite preparation or a fraction thereof to said non-human animal model; and
(ii) determining the level of an internal marker for regulatory T-cell activity in said non-human animal model after said administering, wherein said internal marker is the transcription factor FOXP3 (Scurfin);
wherein an increase in said level of said internal marker after said administering is indicative of treatment efficacy of said helminthic parasite preparation.

6. The method of claim 5, wherein said level of said transcription factor is measured at its protein or mRNA level.

7. The method of claim 5, wherein said level of said marker is found in the lamina propria mononuclear cells of said model.

8. The method of claim 5, wherein said level of said marker is found in the mesenteric lymph nodes of said model.

## Patentansprüche

1. In-vitro-Verfahren zur Prüfung eines Helminthes-Parasitenpräparats, das den Grad einer regulatorischen T-Zell-Aktivität verändert, wobei das Verfahren die folgenden Schritte umfasst:
(a) Gewinnen eines Helminthes-Parasitenpräparats;
(b) In-Kontakt-Bringen des Helminthes-Parasitenpräparats mit einem menschlichen In-vitro-System, das regulatorische T-Zellen enthält; und
(c) Ermitteln des Grads eines internen Markers für regulatorische T-Zell-Aktivität in dem Ziel nach dem In-Kontakt-Bringen, wobei der interne Marker der Transkriptionsfaktor FOXP3 (Scurfin) ist; und
wobei eine Änderung des Grads des internen Markers nach dem In-Kontakt-Bringen anzeigt, dass das Helminthes-Parasitenpräparat eine regulatorische T-Zell-Aktivität verändert.

2. Verfahren nach Anspruch 1, wobei der Grad des Transkriptionsfaktors an seinem Protein- oder mRNA-Grad gemessen wird.

3. Verfahren nach Anspruch 1, wobei die regulatorische T-Zellen-vermittelte Aktivität in den mononukleären Lamina-propria-Zellen des Systems zu finden ist.

4. Verfahren nach Anspruch 1, wobei die regulatorische T-Zellen-vermittelte Aktivität in den Mesenteriallymphknoten des Systems zu finden ist.

5. Verfahren zur Überwachung der Behandlungswirksamkeit eines Helminthes-Parasitenpräparats für eine Autoimmun- oder allergische Erkrankung in dem nicht-menschlichen Tiermodell, das umfasst:
(i) Verabreichen einer Zusammensetzung, die ein Helminthes-Parasitenpräparat oder eine Fraktion davon umfasst, an das nicht-menschliche Tiermodell; und
(ii) Ermitteln des Grads eines internen Markers für regulatorische T-Zell-Aktivität in dem nicht-menschlichen Tiermodell nach der Verabreichung, wobei der interne Marker der Transkriptionsfaktor FOXP3 (Scurfin) ist;
wobei eine Erhöhung in dem Grad des internen Markers nach der Verabreichung die Behandlungswirksamkeit des Helminthes-Parasitenpräparats anzeigt.

6. Verfahren nach Anspruch 5, wobei der Grad des Transkriptionsfaktors an seinem Protein- oder mRNA-Grad gemessen wird.

7. Verfahren nach Anspruch 5, wobei der Grad des Markers in den mononukleären Lamina-propia-Zellen des Modells zu finden ist.

8. Verfahren nach Anspruch 5, wobei der Grad des Markers in den Mesenteriallymphknoten des Modells zu finden ist.

## Revendications

1. Procédé *in vitro* de criblage d'une préparation contre les parasites helminthiques altérant le niveau d'activité des lymphocytes T régulateurs humains, ledit procédé comprenant les étapes consistant à :
(a) obtenir une préparation contre les parasites helminthiques ;
(b) mettre en contact ladite préparation contre les parasites helminthiques avec un système *in vitro* humain contenant des lymphocytes T régulateurs ; et
(c) déterminer le niveau d'un marqueur interne en termes d'activité des lymphocytes T régulateurs dans ladite cible après ladite mise en contact, dans lequel ledit marqueur interne est le facteur de transcription FOXP3 (scurfine) ; et
dans lequel une modification dudit niveau dudit marqueur interne après ladite mise en contact indique que ladite préparation contre les parasites helminthiques altère l'activité des lymphocytes T régulateurs.

2. Procédé selon la revendication 1, dans lequel ledit niveau dudit facteur de transcription est mesuré en termes de teneur en protéine ou en ARNm de celui-ci.

3. Procédé selon la revendication 1, dans lequel ladite activité médiée par les lymphocytes T régulateurs est observée dans les cellules mononucléaires de la lamina propia dudit système.

4. Procédé selon la revendication 1, dans lequel ladite activité médiée par les lymphocytes T régulateurs est observée dans les ganglions lymphatiques mésentériques dudit système.

5. Procédé de contrôle de l'efficacité thérapeutique d'une préparation contre les parasites helminthiques sur une maladie auto-immune ou sur une allergie chez un modèle animal non humain, comprenant les étapes consistant à :
(a) administrer une composition comprenant une préparation contre les parasites helminthiques ou une fraction de celle-ci audit modèle animal non humain ; et
(b) déterminer le niveau d'un marqueur interne en termes d'activité des lymphocytes T régulateurs dans ledit modèle animal non humain après ladite administration, dans lequel ledit marqueur interne est le facteur de transcription FOXP3 (scurfine) ;
dans lequel une augmentation dudit niveau dudit marqueur interne après ladite administration indique que ladite préparation contre les parasites helminthiques a une efficacité thérapeutique.

6. Procédé selon la revendication 5, dans lequel ledit niveau dudit facteur de transcription est mesuré en termes de teneur en protéine ou en ARNm de celui-ci.

7. Procédé selon la revendication 5, dans lequel ledit niveau dudit marqueur est observé dans les cellules mononucléaires de la lamina propia dudit modèle.

8. Procédé selon la revendication 5, dans lequel ledit niveau dudit marqueur est observé dans les ganglions lymphatiques mésentériques dudit modèle.


### SEQUENCE LISTING .

<110> university of Iowa
<120> USE OF PARASITIC BIOLOGICAL AGENTS FOR DISEASES PREVENTION
<130> 27045/2039EP
<140> Not Yet Assigned
   <141> Not Yet Filed
<150> 10/715,659
   <151> 17-November-2003
<160> 9
<170> PatentIn version 3.1
<210> 1
   <211> 42
   <212> DNA
   <213> Artificial sequence
<220>
   <223> synthetic oligonucleotide
<220>
   <221> misc_feature
   <222> (1)..(42)
   <223> synthetic oligonucleotide
<400> 1
   cccaggaaag acagcaacct tttctcacaa ccaggccact tg 42
<210> 2
   <211> 28
   <212> DNA
   <213> Artificial Sequence
<220>
   <223> Synthetic oligonucleotide
<220>
   <221> misc_feature
   <222> (1)..(28)
   <223> synthetic oligonucleotide
<400> 2
   atcctaccca ctgctggcaa atggagtc 28
<210> 3
   <211> 20
   <212> DNA
   <213> Artificial Sequence
<220>
   <223> synthetic oligonucleotide
<220>
   <221> misc_feature
   <222> (1)..(20)
   <223> Synthetic oligonucleotide
<400> 3
   tccatgacgt tcctgacgtt 20
<210> 4
   <211> 20
   <212> DNA
   <213> Artificial sequence
<220>
   <223> synthetic oligonucleotide
<220>
   <221> misc_feature
   <222> (1)..(20)
   <223> Synthetic oligonucleotide
<400> 4 20
   tcctgctgtg caaagtgttc 20
<210> 5
   <211> 20
   <212> DNA
   <213> Artificial sequence
<220>
   <223> Synthetic oligonucleotide
<220>
   <221> misc_feature
   <222> (1)..(20)
   <223> synthetic oligonucleotide
<400> 5
   gagtaaggag gagggggaga 20
<210> 6
   <211> 29
   <212> DNA
   <213> Artificial sequence
<220>
   <223> Synthetic oligonucleotide
<220>
   <221> misc_feature
   <222> (1)..(29)
   <223> synthetic oligonucleotide
<400> 6
   ttgatcttcc cgtaagattc acagcaaca 29
<210> 7
   <211> 28
   <212> DNA
   <213> Artificial sequence
<220>
   <223> Synthetic oligonucleotide
<220>
   <221> misc_feature
   <222> (1)..(28)
   <223> Synthetic oligonucleotide
<400> 7
   tgaagagcta ctgtaatgat cagtcaac 28
<210> 8
   <211> 22
   <212> DNA
   <213> Artificial sequence
<220>
   <223> Synthetic oligonucleotide
<220>
   <221> misc_feature
   <222> (1)..(22)
   <223> synthetic oligonucleotide
<400> 8
   gcaagcttgc aaccttaacc at 22
<210> 9
   <211> 28
   <212> DNA
   <213> Artificial sequence
<220>
   <223> Sythetic oligonucleotide
<220>
   <221> misc_feature
   <222> (1)..(28)
   <223> synthetic oligonucleotide
<400> 9
   tgctttccct ggttaagcag tacagccc 28